# EUROPEAN PATENT APPLICATION

(11) **EP 4 686 760 A1**
(43) Date of publication of application: **04.02.2026**
(21) Application number: 24191750.9
(22) Date of filing: 30.07.2024
(51) Int. Cl.: C12P 19/18, C12N 9/10, C12P 21/00

(54) **SIALYLTRANSFERASES FOR THE SYNTHESIS OF SIALYLATED GLYCANS, GLYCOCONJUGATES AND GLYCOPROTEINS**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to a method for producing α-sialyl-β-D-galactoside saccharides, particularly α-sialyl-(2→3)-β-D-galactoside saccharides and α-sialyl-(2→6)-β-D-galactoside saccharides, from a β-D-galactoside saccharide, a sialic acid donor, and an enzyme with β-galactoside α-sialyltransferase activity. The enzymes with β-galactoside α-sialyltransferase activity used herein do not exhibit catalytic activity towards the hydrolysis of cytidine 5'-monophospho-*N*-acetyl-neuraminic acid to cytidine and N-acetyl-neuraminic acid. The method can be performed *in vitro* and *in vivo* using a genetically engineered cell comprising a nucleic acid encoding said enzyme. Further, said process may be adapted to produce the sialic acid donor CMP-Neu5Ac from low-cost substrates *N*-acetyl-D-glucosamine (GIcNAc), pyruvate, a cytidine phosphate (CMP, CDP or CTP) and polyphosphate in a single reaction mixture with a set of optionally immobilized or optionally co-immobilized enzymes comprising *N*-acylglucoamine 2-epimerase (AGE), an *N*-acetylneuraminate lyase (NAL), an *N*-acylneuraminate cytidylyltransferase (CSS), optional a uridine kinase (UDK), a uridine monophosphate kinase and a polyphosphate kinase 3 (PPK3).

## Description

### Field of the invention

The present invention relates to a method for producing α-sialyl-β-D-galactoside saccharides, particularly α-sialyl-(2→3)-β-D-galactoside saccharides and α-sialyl-(2→6)-β-D-galactoside saccharides, from a β-D-galactoside saccharide, a sialic acid donor, and an enzyme with β-galactoside α-sialyltransferase activity. The enzymes with β-galactoside α-sialyltransferase activity used herein do not exhibit catalytic activity towards the hydrolysis of cytidine 5'-monophospho-*N*-acetyl-neuraminic acid to cytidine and *N*-acetyl-neuraminic acid. The method can be performed *in vitro* and *in vivo* using a genetically engineered cell comprising a nucleic acid encoding said enzyme. Further, said process may be adapted to produce the sialic acid donor CMP-Neu5Ac from low-cost substrates *N*-acetyl-D-glucosamine (GlcNAc), pyruvate, a cytidine phosphate (CMP, CDP or CTP) and polyphosphate in a single reaction mixture with a set of optionally immobilized or optionally co-immobilized enzymes comprising *N*-acylglucoamine 2-epimerase (AGE), an *N*-acetylneuraminate lyase (NAL), an *N*-acylneuraminate cytidylyltransferase (CSS), optional a uridine kinase (UDK), a uridine monophosphate kinase and a polyphosphate kinase 3 (PPK3).

### Background of the invention

Sialylation of oligosaccharides is a critical post-translational modification involving the addition of sialic acid residues to the termini of glycan chains on glycoproteins and glycolipids. This modification plays several important roles in biological systems such as:
**a.** Sialylation affects the stability and half-life of glycoproteins in the bloodstream. Sialic acids can protect glycoproteins from degradation by exoglycosidases and from recognition and removal by the liver. This is particularly important for therapeutic glycoproteins, where sialylation can influence efficacy and dosage.
**b.** Many pathogens, including bacteria and viruses, exploit sialic acid residues to attach to and invade host cells. For example, the influenza virus binds to sialylated glycans on the surface of respiratory epithelial cells to initiate infection. Understanding these interactions is crucial for developing vaccines and antiviral therapies.
**c.** Sialic acids play a significant role in the nervous system. They are abundant in gangliosides, which are essential for brain development and function. Sialylated molecules are involved in synaptic plasticity, neural cell adhesion, and signal transduction processes critical for learning and memory.

Overall, sialylation of oligosaccharides is crucial for numerous physiological processes and has significant implications for health and disease. Thus, ilt would be of great benefit to be able to efficiently sialylate oligosaccharides, glycoproteins and glycolipids.

Sialylation occurs in two stages, the first is to activate sialic acid and the second is to transfer it to the target molecule. The activation step is catalyzed by the enzyme CMP-Neu5Ac synthetase (CSS, or CNS).

*N*-acetylneuraminic acid (Neu5Ac) is a sialic acid and a nine-carbon (C-9) acidic monosaccharide that occurs naturally at the end of sugar chains attached to the surfaces of cells and soluble proteins. In the human body, the highest concentration of *N*-acetylneuraminic acid occurs in the brain where it participates as an integral part of ganglioside structure in synaptogenesis and neural transmission. Human milk also contains a high concentration of sialic acid attached to the terminal end of free oligosaccharides, in particular, galactosyl-ceramide. In certain pathologies, e.g. in aggressive tumors of neuro-ectodemic origin, Neu5Ac is over-expressed on cell surface.

Certain strains of bacteria contain large amounts of *N*-acetylneuraminic acid in their capsular polysaccharide. For example, *Neisseria meningitidis* serogroup B capsular polysaccharide is a linear homopolymer of sialic acid consisting of approximately 200 repeated units of α(2-8)-linked N-acetyl neuraminic acid (Bhattacharjee A. K. et al., J. Biol. Chem. 1975, 250, pp.1926-1932.) This homopolymer is not restricted to *N. meningitidis* serogroup B since it is also present in the capsule of *Escherichia coli* K1, a pathogen that causes meningitis in newborn children, in *Pasteurella haemolytica A2,* an important veterinary pathogen and also in *Moraxella non-liquefaciens,* a non-pathogenic microorganism common in nasal graves.

*N*-acetylneuraminic acid rarely occurs free in nature. They are more commonly present as components of oligosaccharide chains of mucins, glycoproteins, and glycolipids. They usually occupy terminal, non-reducing positions of oligosaccharide chains of complex carbohydrates on outer and inner membrane surfaces in various linkages, mainly to galactose, *N*-acetylgalactosamine, and other sialic acid moieties, where they are highly exposed and functionally important.

Sialyltransferase may be responsible for the synthesis of the sequence Neu5Ac-α-2,3-Gal-β-1,3-GalNAc-, found on sugar chains O-linked to Thr or Ser and also as a terminal sequence on certain gangliosides. These enzymes catalyze sialyltransfer reactions during glycosylation, and are type II membrane proteins.

CMP-Neu5Ac is a donor substrate for sialyltransferases which attach sialic acid to acceptor hydroxyl groups in various biopolymers including polysialic acids, glycolipids and glycoproteins (Tsuji, 1996).

Human sialyltransferases but normally not other mammalian sialyltransferases, and sialyltransferases of bacterial origin often exhibit pronounced hydrolase activity toward their nucleotide-activated donor substrate, e.g. CMP-Neu5Ac. Particularly sialyltransferases of family GT-80 are effective CMP-Neu5Ac hydrolases in the absence, but also in the presence of an acceptor substrate (Schmölzer et al. FEBS Letters 588 (2014) 2978-2984).

The international patent application WO 2019/020707 A1 discloses sialyltransferases expressed in a genetically modified cell, which are capable of producing complex sialylated human milk oligosaccharides (HMOs). The sialyltransferases disclosed therein, however, only produce minor amounts of the complex sialylated HMOs, with high by-product formation.

The international patent application WO 2023/247537 A1 discloses a production method using genetically modified cells expressing sialyltransferases for *in vivo* synthesis of sialyl-lacto-N-neotetraose (LST-c).

The international patent application WO 2019/126749 A1 discloses α2-6-sialyltransferase variants of *P*. *damselae* (Pd2,6ST) having improved α2-6-specific sialidase activity as compared to the native α2-6-sialyltransferases.

However, side-activities of the sialyltransferases in genetically modified microorganisms which are used for producing sialylated oligosaccharides in whole cell fermentation may affect the ability of the microorganism to grow robustly even in the absence of substrate, thereby resulting in poor yields of the sialylated saccharide.

Thus, there is a high demand for silalyltransferases that allow efficient production of specific sialylated oligosaccharides without formation of by-products.

The objective of the present invention is solved by the teaching of the independent claims. Further advantageous features, aspects and details of the invention are evident from the dependent claims, the description, the figures, and the examples of the present application.

### Description of the invention

By domain analysis the inventors identified new β-galactoside-α-sialyltransferase sequences from multiple bacteria, which could be successfully produced recombinantly in *E*. *coli* and which exhibits a high reactivity and specificity towards certain β-D-galactoside oligosaccharides, particularly human milk oligosaccharides comprising a β-D-galactoside unit. More importantly, said β-galactoside-α-sialyltransferase enzymes do not exhibit catalytic activity towards the hydrolysis (hydrolase activity) of cytidine 5'-monophospho-*N*-acetyl-neuraminic acid to cytidine and *N*-acetyl-neuraminic acid.

Thus, the present invention is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9;
B) mixing said solution and said enzyme and reacting a resulting solution to produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence with at least 85% identity to SEQ ID NOs: 4 - 9 as set forth in SEQ ID NOs: 4 - 9;
B) mixing said solution and said enzyme and reacting a resulting solution to
   produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence with at least 90% identity to SEQ ID NOs: 4 - 9 as set forth in SEQ ID NOs: 4 - 9;
B) mixing said solution and said enzyme and reacting a resulting solution to
   produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence with at least 95% identity to SEQ ID NOs: 4 - 9 as set forth in SEQ ID NOs: 4 - 9;
B) mixing said solution and said enzyme and reacting a resulting solution to produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence with at least 97% identity to SEQ ID NOs: 4 - 9 as set forth in SEQ ID NOs: 4 - 9;
B) mixing said solution and said enzyme and reacting a resulting solution to produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence with at least 98% identity to SEQ ID NOs: 4 - 9 as set forth in SEQ ID NOs: 4 - 9;
B) mixing said solution and said enzyme and reacting a resulting solution to
   produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence with at least 99% identity to SEQ ID NOs: 4 - 9 as set forth in SEQ ID NOs: 4 - 9;
B) mixing said solution and said enzyme and reacting a resulting solution to produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the α-sialyl-β-D-galactoside saccharide is an α-sialyl-(2→6)-β-D-galactoside saccharide and the enzyme with β-galactoside α-sialyltransferase activity is an enzyme with β-galactoside α-2,6-sialyltransferase activity consisting of an amino acid sequence as set forth in SEQ ID NOs: 4 - 5 or an amino acid sequence with at least 80% identity to SEQ ID NOs: 4-5.

An alternative preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the α-sialyl-β-D-galactoside saccharide is an α-sialyl-(2→3)-β-D-galactoside saccharide and the enzyme with β-galactoside α-sialyltransferase activity is an enzyme with β-galactoside α-2,3-sialyltransferase activity consisting of an amino acid sequence as set forth in SEQ ID NOs: 6 - 9 or an amino acid sequence with at least 80% identity to SEQ ID NOs: 6-9.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the enzyme with β-galactoside α-sialyltransferase activity does not catalyse the hydrolysis of cytidine 5'-monophospho-*N*-acetyl-neuraminic acid to cytidine and *N*-acetyl-neuraminic acid.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4, 7, and 8 or an amino acid sequence with at least 80% identity to SEQ ID NOs: 4, 7, and 8;
B) mixing said solution and said enzyme and reacting a resulting solution to produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4, 7, and 8 or an amino acid sequence with at least 90% identity to SEQ ID NOs: 4, 7, and 8;
B) mixing said solution and said enzyme and reacting a resulting solution to produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4, 7, and 8 or an amino acid sequence with at least 95% identity to SEQ ID NOs: 4, 7, and 8;
B) mixing said solution and said enzyme and reacting a resulting solution to produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4, 7, and 8 or an amino acid sequence with at least 97% identity to SEQ ID NOs: 4, 7, and 8;
B) mixing said solution and said enzyme and reacting a resulting solution to produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4, 7, and 8 or an amino acid sequence with at least 98% identity to SEQ ID NOs: 4, 7, and 8;
B) mixing said solution and said enzyme and reacting a resulting solution to produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4, 7, and 8 or an amino acid sequence with at least 99% identity to SEQ ID NOs: 4, 7, and 8;
B) mixing said solution and said enzyme and reacting a resulting solution to produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4, 7, and 8;
B) mixing said solution and said enzyme and reacting a resulting solution to produce the α-sialyl-β-D-galactoside saccharide.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the β-D-galactoside saccharide is a glycopeptide, a glycoprotein, a glycolipid, a glycan or a human milk oligosaccharide (HMO).

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the β-D-galactoside saccharide comprises one or more terminal β-D-galactose residues,

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the α-sialyl-β-D-galactoside saccharide is a sialylated human milk oligosaccharide selected from 6'-siallylactose, 3'-siallylactose, sialyllacto-N-tetraose a (LSTa), sialyllacto-N-neotetraose d (LSTd), sialyllacto-N-neotetraose c (LSTc), sialyllacto-N-tetraose b (LSTb) and disialyllacto-N-tetraose (DSLNT).

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the β-D-galactoside saccharide is a glycoprotein bearing A2G2 (GlcNAc₂Man₃GlcNAc₂Gal₂).

An alternative preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the α-sialyl-(2→6)-β-D-galactoside saccharide is a sialylated monoclonal antibody.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the sialic acid donor is a sugar nucleotide, preferably CMP-Neu5Ac.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the solution has a pH between 6 and 9.

A preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein step B) is carried out at a reaction temperature between 25 °C and 50 °C.

A preferred embodiment is directed to an *in vitro* method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide.

In an alternative embodiment, the sialic acid donor CMP-Neu5Ac is formed *in situ* from acetyl-D-glucosamine, pyruvate, polyphosphate, CMP, and adenosine 5'-triphosphate (ATP) and a set of enzymes. Thus, the present invention is also directed to an *in vitro* method for producing an α-sialyl-β-D-galactoside saccharide, comprising
A') providing a solution comprising a β-D-galactoside saccharide, optionally a sialic acid donor, *N*-acetyl-D-glucosamine, pyruvate, polyphosphate, catalytic amounts of i) cytidine 5'-monophosphate, ii) cytidine 5'-diphosphate or iii) cytidine 5'-triphosphate, and catalytic amounts of i) adenosine 5'-monophosphate, adenosine 5'-diphosphate, or adenosine 5'-triphosphate, and
   a set of enzymes comprising an N-acylglucosamine 2-epimerase (AGE, EC 5.1.3.8), an N-acetylneuraminate lyase (NAL, EC 4.1.3.3), an N-acetylneuraminate cytidylyltransferase (CSS, EC 2.7.7.43), optional a uridine kinase (UDK, EC 2.7.1.48), a uridine monophosphate kinase (URA6, EC 2.7.4.22), a polyphosphate kinase 3 (PPK3, EC2.7.4.1), and an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said set of enzymes and reacting a resulting solution to produce the α-sialyl-β-D-galactoside saccharide.

A preferred embodiment is directed to an *in vitro* method for producing an α-sialyl-β-D-galactoside saccharide, comprising
A) providing a solution comprising a β-D-galactoside saccharide, a sialic acid donor, *N*-acetyl-D-glucosamine, pyruvate, polyphosphate, catalytic amounts of
   i) cytidine 5'-monophosphate, ii) cytidine 5'-diphosphate or iii) cytidine 5'-triphosphate, and catalytic amounts of i) adenosine 5'-monophosphate, adenosine 5'-diphosphate, or adenosine 5'-triphosphate, and
      a set of enzymes comprising an N-acylglucosamine 2-epimerase (AGE) as set forth in SEQ ID NO: 10, an N-acetylneuraminate lyase (NAL) as set forth in SEQ ID NO: 11, an N-acetylneuraminate cytidylyltransferase (CSS) as set forth in SEQ ID NO: 12, optional a uridine kinase (UDK) as set forth in SEQ ID NO: 13, a uridine monophosphate kinase as set forth in SEQ ID NO: 14, a polyphosphate kinase 3 (PPK3) as set forth in SEQ ID NO: 15, and an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,.

A preferred embodiment is directed to an *in vitro* method for producing an α-sialyl-β-D-galactoside saccharide, comprising
A) providing a solution comprising a β-D-galactoside saccharide, a sialic acid donor, *N*-acetyl-D-glucosamine, pyruvate, polyphosphate, catalytic amounts of
   i) cytidine 5'-monophosphate, ii) cytidine 5'-diphosphate or iii) cytidine 5'-triphosphate, and catalytic amounts of i) adenosine 5'-monophosphate, adenosine 5'-diphosphate, or adenosine 5'-triphosphate, and
      a set of enzymes comprising an N-acylglucosamine 2-epimerase (AGE), an N-acetylneuraminate lyase (NAL), an N-acetylneuraminate cytidylyltransferase (CSS), optional a uridine kinase (UDK), a uridine monophosphate kinase, a polyphosphate kinase 3 (PPK3), and an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the set of enzymes is co-immobilized together with the β-galactoside α-2,6-sialyltransferase on a solid support.

An alternative embodiment is directed to an *in vivo* method for producing an α-sialyl-β-D-galactoside saccharide, comprising
A') providing a solution comprising a β-D-galactoside saccharide, optionally a sialic acid donor, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside saccharide,
   wherein the solution is a fermentation broth further comprising at least one carbon source.

### Detailed description of the invention

### Definitions

As used herein, the term "**sialyltransferase**" is an enzyme of the GT family that plays an integral role in the biosynthesis of Neu5Ac containing oligosaccharides and glycoconjugates. Generally in glycosylation reactions catalyzed by STs, the sugar nucleotide donor is cytidine 5'-monophosphate Neu5Ac (CMP-Neu5Ac), and the acceptor is an oligosaccharide or glycoconjugate terminated by a galactose (Gal), *N*-acetylgalactosamine (GalNAc), or other Neu5Ac residue. STs are classified based on the position of the glycosyl acceptor that Neu5Ac is transferred to. In humans, these are ST3, ST6, and ST8, which form an α-glycosidic bond between the C2 atom of Neu5Ac and the 3'-, 6'-, or 8'-hydroxyl group of the acceptor, respectively. Preferably, "**sialyltransferase**" is selected from beta-galactosamide alpha-2,6-sialyltransferase (EC 2.4.99.1), alpha-N-acetylgalactosaminide alpha-2,6-sialyltransferase (EC 2.4.99.3), beta-galactoside alpha-2,3-sialyltransferase (EC 2.4.99.4), N-acetyllactosaminide alpha-2,3-sialyltransferase (EC 2.4.99.6), alpha-N-acetyl-neuraminide alpha-2,8-sialyltransferase (EC 2.4.99.8); and lactosylceramide alpha-2,3-sialyltransferase (EC 2.4.99.9). These enzymes use the CMP-Neu5Ac as a sialic acid donor.

As used herein, the term **"sialic acid donor"** refers to a molecule comprising a sialic acid (*N*-acetylneuraminic acid) moiety and which is a substrate of a sialyltransferase, specifically a beta-galactosamide alpha-2,6-sialyltransferase. Examples of sialic acid donors are sialic acid nucleotides, such as CMP-Neu5Ac.

As used herein, the term **"N-acylglucosamine 2-epimerase** (GlcNAc 2-epimerase, ACE)" refers to an enzyme having an active domain that catalyzes the epimerization of N-acyl-D-glucosamine to N-acyl-D-mannosamine as follows:

| | |
|---|---|
| N-acyl-D-glucosamine | N-acyl-D-mannosamine |

Hence, this enzyme has one substrate, N-acyl-D-glucosamine, and one product, N-acyl-D-mannosamine. This enzyme belongs to the family of isomerases, specifically to those racemases and epimerases acting on carbohydrates and derivatives.

The N-acylglucosamine 2-epimerase belongs to the EC class 5.1.3.8. The N-acylglucosamine 2-epimerase has also the following synonyms: N-acyl-D-glucosamine 2-epimerase, acylglucosamine 2-epimerase, and N-acetylglucosamine 2-epimerase. This enzyme participates in aminosugar metabolism. It employs one cofactor, ATP.

As used herein, the term "***N*-acetylglucosamine deacetylase"** refers to an enzyme having an active domain catalyzing the following reaction:

N-acetyl-D-glucosamine + H₂O D-glucosamine + acetate

This enzymatic reaction is reversible and thus in this invention, the N-acetylglucosamine deacetylase is used for producing N-acetyl-D-glucosamine (GlcNAc) by pushing the equilibrium to production of GlcNAc. Therefore, in the present invention this enzyme uses two substrates D-glucosamine and acetate for producing N-acetyl-D-glucosamine.

This enzyme belongs to the family of hydrolases, those acting on carbon-nitrogen bonds other than peptide bonds, specifically in linear amides. The N-acetylglucosamine deacetylase belongs to the EC class EC 3.5.1.33. The N-acetylglucosamine deacetylase has also the following synonyms: N-acetyl-D-glucosamine amidohydrolase, acetylaminodeoxyglucose acetylhydrolase, and N-acetyl-D-glucosaminyl N-deacetylase.

As used herein, the term **"N-acetylneuraminate lyase** (NAL)" refers to a polypeptide having active domain catalyzing the following reaction:

N-acetylneuraminate N-acetyl-D-mannosamine + pyruvate

This enzymatic reaction is reversible and thus in this invention, the N-acetylneuraminase lyase is used for producing CMP-Neu5Ac by pushing the equilibrium to production of Neu5Ac. Therefore, in the present invention this enzyme uses two substrates N-acetyl-D-mannosamine and pyruvate for producing N-acetylneuraminate.

This enzyme belongs to the family of lyases, specifically the oxo-acid-lyases, which cleave carbon-carbon bonds. The N-acetylneuraminate lyase belongs to the EC class EC 4.1.3.3. The N-acetylneuraminate lyase has also the following synonyms: N-acetylneuraminate pyruvate-lyase (N-acetyl-D-mannosamine-forming). Other names in common use include N-acetylneuraminic acid aldolase, acetylneuraminate lyase, sialic aldolase, sialic acid aldolase, sialate lyase, N-acetylneuraminic aldolase, neuraminic aldolase, N-acetylneuraminate aldolase, neuraminic acid aldolase, N-acetylneuraminic acid aldolase, neuraminate aldolase, N-acetylneuraminic lyase, N-acetylneuraminic acid lyase, NPL, NALase, NANA lyase, acetylneuraminate pyruvate-lyase, and N-acetylneuraminate pyruvate-lyase. This enzyme participates in aminosugar metabolism.

As used herein, the term **"N-acylneuraminate cytidylyltransferase** (CSS)" refers to a polypeptide having an active domain catalyzing the reaction of cytidine 5'-triphosphate (CTP) with N-acetyl-D-neuraminic acid and producing CMP-*N-*acetyl- D-neuraminic acid (CMP-Neu5Ac).

The *N*-acylneuraminate cytidylyltransferase has also the following synonyms: CMP-sialate pyrophosphorylase, CMP-sialate synthase, cytidine 5'-monophosphosialic acid synthetase, CMP-Neu5Ac synthetase (CNS), CMP-NeuAc synthetase, acylneuraminate cytidyltransferase, CMP-*N*-acetylneuraminate synthetase, CMP-*N*-acetylneuraminate synthase, CMP-*N*-acetylneuraminic acid synthase, CMP-NANA synthetase, CMP-sialate synthetase, CMP-sialic synthetase, cytidine 5'-monophospho-*N*-acetylneuraminic acid synthetase, cytidine 5-monophosphate *N*-acetylneuraminic acid synthetase, cytidine monophosphosialic acid synthetase, cytidine monophosphoacetylneuraminic synthetase, cytidine monophosphosialate pyrophosphorylase, cytidine monophosphosialate synthetase, and acetylneuraminate cytidylyltransferase.

The *N*-acylneuraminate cytidylyltransferase belongs to the EC class 2.7.7.43. The *N*-acylneuraminate cytidylyltransferase catalyzes the following reaction:

Neu5Ac + CTP → CMP-Neu5Ac + PPi

N-acylneuraminate cytidylyltransferase is obtained from microorganisms including *Cricetulus griseus, Escherichia coli, Haemophilus ducreyi, Haemophilus influenza, Hungateiclostridium thermocellum, Mannheimia haemolytica, Neisseria meningitidis, Oncorhynchus mykiss, Pasteurella haemolytica A2, Pelophylax esculentus, Photobacterium leiognathi, Rattus norvegicus, Streptococcus agalactiae,* and *Sus scrota;* mouse, rat, calf and rainbow trout.

Mutants of *Neisseria meningitidis* CSS have at least one of the following mutations: Q104A, R165A, Q166A, N175A, Y179A, F192A, F193A.

Kinases are enzymes which form a part of the family of the phosphotransferases. Kinases are enzymes that catalyze the transfer of phosphate groups from high-energy, phosphate-donating molecules to specific substrates. This process is known as phosphorylation, where the substrate gains a phosphate group and the high-energy nucleotide, e.g. adenosine triphosphate (ATP), molecule donates a phosphate group. This transesterification produces a phosphorylated substrate and ADP.

As used herein, the term **"uridine kinase"** refers to a polypeptide having an active domain catalyzing the reaction of uridine to uridine 5'-monophosphate in the presence of adenosine triphosphate (ATP) as follows:

ATP + uridine ↔ ADP + UMP

Thus, the two substrates of this enzyme are ATP and uridine, whereas its two products are ADP and UMP.

In addition, it was found that the uridine kinase is able to catalyze the reaction of cytidine to cytidine 5'-monophosphate in the presence of adenosine triphosphate as follows:
Cytidine + ATP 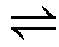 CMP + ADP

This enzyme belongs to the family of transferases, specifically those transferring phosphorus-containing groups (phosphotransferases) with an alcohol group as acceptor. The uridine kinase belongs to the EC class 2.7.1.48. Other names in common use include pyrimidine ribonucleoside kinase, uridine-cytidine kinase, uridine kinase (phosphorylating), and uridine phosphokinase. This enzyme participates in pyrimidine metabolism.

As used herein, the term **"uridine monophosphate (UMP) kinase"** or refers to a polypeptide having an active domain catalyzing the reaction of uridine 5'-monophosphate to uridine 5'-diphosphate in the presence of adenosine triphosphate. The uridine monophosphate kinase belongs to the EC class 2.7.4.22. The uridine monophosphate kinase catalyzes the following reaction:

UMP + ATP 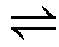 UDP + ADP

In addition, it was found that the uridine monophosphate (UMP) kinase is able to catalyze the reaction of cytidine monophosphate to cytidine 5'-diphosphate in the presence of adenosine triphosphate as follows:

CMP + ATP 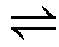 CDP + ADP

This enzyme belongs to the family of transferases, specifically those transferring phosphorus-containing groups (phosphotransferases) with a phosphate group as acceptor. Other names in common use include uridylate kinase, UMPK, uridine monophosphate kinase, PyrH, UMP-kinase, and SmbA. This enzyme participates in pyrimidine metabolism.

As used herein, the term **"polyphosphate"** refers to any salts containing several P-O-P bonds generated by corner sharing of six or more phosphate (PO₄) tetrahedral, leading to the formation of long chains. The term "PolyPₙ" is synonymously used, wherein n represents average chain length of the number of phosphate residues, *e.g.* PolyP₂₅ refers to a polyphosphate having about 25 phosphate residues and PolyP₁₄ refers to a polyphosphate having about 14 phosphate residues.

As used herein, the term **"polyphosphate kinase"** refers to a polypeptide having polyphosphate kinase activity, i.e. a polyphosphate kinase catalyzes the following reactions:

NMP + polyphosphate (n+1) 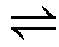 NDP + polyphosphate(n)

NDP + polyphosphate (n+1) 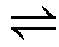 NTP + polyphosphate(n)

with N being a nucleotide such as guanosine, adenosine, uridine etc. and NMP being nucleoside monophosphate, NDP being nucleoside diphosphate and NTP being nucleoside triphosphate.

In case of uridine the polyphosphate kinase catalyzes the following reaction:

ADP + polyphosphate (n+1) 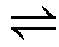 ATP + polyphosphate(n)

AMP + polyphosphate (n+1) 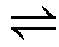 ADP + polyphosphate(n)

UDP + polyphosphate (n+1) 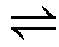 UTP + polyphosphate(n)

The polyphosphate kinase belongs to the EC class 2.7.4.1. Representatives of the polyphosphate kinase enzyme used in the inventive methods described herein include but are not limited to polyphosphate kinase 1 (PPK1), polyphosphate kinase 2 (PPK2), 2-domain polyphosphate kinase 2 (2D-PPK2) and 1-domain polyphosphate kinase 2 (1D-PPK2) and polyphosphate kinase 3 (PPK3).

As used herein, the term **"pyrophosphatase"** refers to a polypeptide having pyrophosphatase activity, *i.e.* a polypeptide that catalyzes the following reaction:

PPi + H₂O 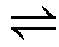 2 Pi

wherein PPi refers to pyrophosphate and Pi to phosphate.

The pyrophosphatase belongs to EC classes 3.6.1.1. In this context, the term "diphosphatase" refers to a pyrophosphatase polypeptide which catalyzes the hydrolysis of diphosphate to phosphate.

As used herein, **"saccharide"** refers to but not restricted to monosaccharide, disaccharide, trisaccharide, tetrasaccharide, pentasaccharide, hexasaccharide, heptasaccharide, octasaccharide, oligosaccharide, glycan and polysaccharide. The saccharide comprises preferably at least one of monosaccharide units selected from:
D-Arabinose, D-Lyxose, D-Ribose, D-Xylose, L-Arabinose, L-Lyxose, L-Ribose, L-Xylose, D-Ribulose, D-Xylulose, L-Ribulose, L-Xylulose, D-Deoxyribose, L-Deoxyribose, D-Erythrose, D-Threose, L-glycero-D-manno-Heptose, D-glycero-D-manno-Heptose, D-Allose, D-Altrose, D-Glucose, D-Mannose, D-Gulose, D-Idose, D-Galactose, D-Talose, D-psicose, D-fructose, D-sorbose, D-tagatose, 6-Deoxy-L-altrose, 6-Deoxy-D-talose, D-Fucose, L-Fucose, D-Rhamnose, L-Rhamnose, D-Quinovose, Olivose, Tyvelose, Ascarylose, Abequose, Paratose, Digitoxose, Colitose, D-Glucosamine, D-Galactosamine, D-Mannosamine, D-Allosamine, I-Altrosamine, D-Gulosamine, L-Idosamine, D-Talosamine, N-Acetyl-d-glucosamine, N-Acetyl-D-galactosamine, N-Acetyl-D-mannosamine, N-Acetyl-D-allosamine, N-Acetyl-L-altrosamine, N-Acetyl-D-gulosamine, N-Acetyl-L-idosamine, N-Acetyl-D-talosamine, N-Acetyl-D-fucosamine, N-Acetyl-L-fucosamine, N-Acetyl-L-rhamnosamine, N-Acetyl-D-quinovosamine, D-Glucuronic acid, D-Galacturonic acid, D-Mannuronic acid, D-Alluronic acid, L-Altruronic acid, D-Guluronic acid, L-Guluronic acid, L-Iduronic acid, D-Taluronic acid, Neuraminic acid, N-Acetylneuraminic acid, N-Glycolylneuraminic acid, 3-Deoxy-D-manno-octulosonic Acid (KDO), 3-Deoxy-D-glycero-D-galacto-2-nonulosonic Acid (KDN), Apiose, Bacillosamine, Thevetose, Acofriose, Cymarose, Muramic acid, N-Acetylmuramic acid, N-Glycolylmuramic acid, 3-Deoxy-lyxo-heptulosaric acid, Ketodeoxyoctonic acid, and Ketodeoxynononic acid. Preferably the monosaccharide or monosaccharide unit belongs to the following group of α- and β-D/L-carbohydrates comprising or consisting of:
α-D-ribopyranose, α-D-arabinopyranose, α-D-xylopyranose, α-D-lyxopyranose, α-D-allopyranose, α-D-altropyranose, α-D-glucopyranose, α-D-mannopyranose, α-D-glucopyranose, α-D-idopyranose, α-D-galactopyranose, α-D-talopyranose, α-D-psicopyranose, α-D-fructopyranose, α-D-sorbopyranose, α-D-tagatopyranose, α-D-ribofuranose, α-D-arabinofuranose, α-D-xylofuranose, α-D-lyxofuranose, α-D-Allofuranose, α-D-Altrofuranose, α-D-Glucofuranose, α-D-Mannofuranose, α-D-gulofuranose, α-D-idofuranose, α-D-galactofuranose, α-D-talofuranose, α-D-psicofuranose, α-D-fructofuranose, α-D-sorbofuranose, α-D-tagatofuranose, α-D-xylulofuranose, α-D-ribulofuranose, α-D-threofuranose, α-D-rhamnopyranose, α-D-erythrofuranose, α-D-glucosamine, α-D-N-acetyl-glucosamine, α-D-glucopyranuronic acid, α-D-galactosamine, α-D-N-acetyl-galactosamine, α-D-mannosamine, α-D-N-acetyl-mannosamine, α-D-neuraminic acid, α-D-N-acetylneuraminic acid, α-D-N-Glycolylneuraminic acid, α-3-Deoxy-D-manno-octulosonic acid (KDO), α-3-Deoxy- D-glycero-D-galacto-2-nonulosonic Acid (KDN),
β-D-ribopyranose, β-D-arabinopyranose, β-D-xylopyranose, β-D-lyxopyranose, β-D-allopyranose, β-D-altropyranose, β-D-glucopyranose, β-D-mannopyranose, β-D-glucopyranose, β-D-idopyranose, β-D-galactopyranose, β-D-talopyranose, β-D-psicopyranose, β-D-fructopyranose, β-D-sorbopyranose, β-D-tagatopyranose, β-D-ribofuranose, β-D-arabinofuranose, β-D-xylofuranose, β-D-lyxofuranose, β-D-rhamnopyranose, β-D-allofuranose, β-D-altrofuranose, β-D-glucofuranose, β-D-mannofuranose, β-D-gulofuranose, β-D-idofuranose, β-D-galactofuranose, β-D-talofuranose, β-D-psicofuranose, β-D-fructofuranose, β-D-sorbofuranose, β-D-tagatofuranose, β-D-xylulofuranose, β-D-ribulofuranose, β-D-threofuranose, P-D-erythrofuranose, β-D-glucosamine, β-D-N-acetyl-glucosamine, β-D-glucopyranuronic acid, β-D-galactosamine, β-D-N-acetyl-galactosamine, β-D-mannosamine, β-D-N-acetyl-mannosamine, β-D-neuraminic acid, β-D-N-acetylneuraminic acid, β-D-N-glycolylneuraminic acid, β-3-Deoxy-D-manno-octulosonic acid (KDO), β-3-Deoxy- D-glycero-D-galacto-2-nonulosonic Acid (KDN),
α-L-ribopyranose, α-L-arabinopyranose, α-L-xylopyranose, α-L-lyxopyranose, α-L-allopyranose, α-L-altropyranose, α-L-glucopyranose, α-L-mannpyranose, α-L-glucopyranose, α-L-idopyranose, α-L-galactopyranose, α-L-talopyranose, α-L-psicopyranose, α-L-fructopyranose, α-L-sorbopyranose, α-L-tagatopyranose, α-L-rhamnopyranose, α-L-ribofuranose, α-L-arabinofuranose, α-L-xylofuranose, α-L-lyxofuranose, α-L-Allofuranose, α-L-Altrofuranose, α-L-Glucofuranose, α-L-Mannofuranose, α-L-gulofuranose, α-L-idofuranose, α-L-galactofuranose, α-L-talofuranose, α-L-psicofuranose, α-L-fructofuranose, α-L-sorbofuranose, α-L-tagatofuranose, α-L-xylulofuranose, α-L-ribulofuranose, α-L-threofuranose, α-L-erythrofuranose, α-L-glucosamine, α-L-glucopyranuronic acid,
β-L-ribopyranose, β-L-arabinopyranose, β-L-xylopyranose, β-L-lyxopyranose, β-L-allopyranose, β-L-altropyranose, β-L-glucopyranose, β-L-mannpyranose, P-L-glucopyranose, β-L-idopyranose, β-L-galactopyranose, β-L-taloopyranose, β-L-psicopyranose, β-L-fructopyranose, β-L-sorbopyranose, β-L-tagatopyranose, β-L-ribofuranose, β-L-arabinofuranose, β-L-xylofuranose, β-L-lyxofuranose, β-L-allofuranose, β-L-altrofuranose, β-L-glucofuranose, β-L-mannofuranose, β-L-gulofuranose, β-L-idofuranose, β-L-galactofuranose, β-L-talofuranose, β-L-psicofuranose, β-L-fructofuranose, β-L-sorbofuranose, β-L-tagatofuranose, β-L-xylulofuranose, β-L-ribulofuranose, β-L-threofuranose, β-L-erythrofuranose, β-L-glucosamine, β-L-glucopyranuronic acid, and β-L-rhamnopyranose.

The saccharides are further optionally modified to carry amide, carbonate, carbamate, carbonyl, thiocarbonyl, carboxy, thiocarboxy, ester, thioester, ether, epoxy, hydroxyalkyl, alkylenyl, phenylene, alkenyl, imino, imide, isourea, thiocarbamate, thiourea and/or urea moieties.

Preferably, **"saccharide"** is a human milk oligosaccharide including lactose, *N-*acetyl-lactosamine, lacto-*N*-biose, 2'-fucosyllactose, 3-fucosyllactose (3-FL), lacto-*N*-tetraose (LNT), lacto-*N*-neotetraose (LNnT), difucosyllactose(DiFL), lacto-*N-*triose II (LNT-II), lacto-*N*-fucopentaose I (LNFP I), lacto-*N*-fucopentaose III (LNFP III), lacto-*N*-fucopentaose V (LNFPV).

As used herein, the term "**α-sialyl-(2→6)-β**-**D-galactoside saccharide"** refers to a saccharide having one or more α-sialyl-(2→6)-β**-**D-galactosyl moieties.

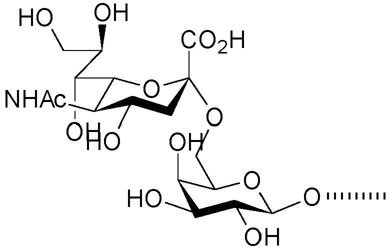

Exemplary α-sialyl-(2→6)-β-D-galactoside saccharides are sialylated HMOS, such as 6'-siallylactose, sialyllacto-N-tetraose c (LSTc) and disialyllacto-N-tetraose (DSLNT), as well as sialylated glycans such as A2G2S1, A2G2S2 or sialylated monoclonal antibodies.

As used herein, the term **"α-sialyl-(2→3)-β-D-galactoside saccharide"** refers to a saccharide having one or more α-sialyl-(2→3)-β-D-galactosyl moieties.

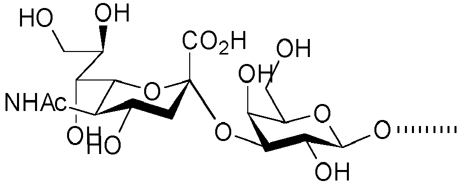

Exemplary α-sialyl-(2→3)-β-D-galactoside saccharides are sialylated HMOS, such as sialyllacto-N-tetraose a (LSTa), sialyllacto-N-neotetraose d (LSTd) or sialylated monoclonal antibodies.

As used herein, the term "**D-galactoside saccharide"** refers to a saccharide having one or more β-D-galactosyl moieties. The β-D-galactosyl moiety acts as an acceptor for the inventive sialyltransferase. The β-D-galactosyl moiety may be located at the end of the saccharide (terminal) or positioned within the saccharide chain.

As used herein, the term **"glycopeptide"** refers to a peptide that contains carbohydrate moieties covalently attached to the side chains of the amino acid residues that constitute the peptide. The carbohydrate moieties form side chains and are either O-glycosidic connected to the hydroxy group of a serine or threonine residue or N-glycosidic connected to the amido nitrogen of an asparagine residue.

As used herein, the term **"glycoprotein"** refers to a polypeptide that contains carbohydrate moieties covalently attached to the side chains of the amino acid residues that constitute the polypeptide. The carbohydrate moieties form side chains and are either O-glycosidic connected to the hydroxy group of a serine or threonine residue or N-glycosidic connected to the amido nitrogen of an asparagine residue.

As used herein, the term **"glycolipid"** refers to a compound containing one or more monosaccharide moieties bound by a glycosidic linkage to a hydrophobic moiety. Glycolipids consist of monoglycosyldiacylglycerol (MGDG), diglycosyldiacylglycerol (DGDG), trimethyl-beta-alaninediacylglycerol, and sulphaquinovosyldiacyl-glycerol. Different glycolipid classes exist having various possible backbone molecular structures such as acylglycerols, sphingoids, ceramides (*N*-acylsphingoids), and sterols.

In particular, a ganglioside is a molecule composed of a glycosphingolipid (ceramide and oligosaccharide) with one or more N-acetylneuraminic acid, Neu5Ac) linked on the sugar chain. Types of ganglioside includes LM1, GM1, GM1b, and GM2 which comprise one N-acetylneuraminic acid; GD1a, GalNAc-GD1a, GD1b, GD2, and GD3 which comprise two N-acetylneuraminic acids; GT1a, and GT3 which comprise three N-acetylneuraminic acids; and GQ1b which comprises four N-acetylneuraminic acids.

As used herein, the term **"protein"** refers to a polypeptide that contains or lacks of carbohydrate moieties covalently attached to the side chains of the amino acid residues that constitute the polypeptide including aglycosylated proteins and glycosylated proteins.

As used herein, the term **"peptide"** refers to a peptide that contains or lacks of carbohydrate moieties covalently attached to the side chains of the amino acid residues that constitute the peptide, including aglycosylated peptides and glycosylated peptides.

The term **"solid support"** as used herein refers to an insoluble, functionalized, material to which enzymes or other reagents may be attached or immobilized, directly or via a linker bearing an anchoring group, allowing enzymes to be readily separated (by washing, filtration, centrifugation, etc.) from excess reagents, soluble reaction products, by-products, or solvents. A solid support can be composed of organic polymers such as polystyrene, polyethylene, polypropylene, polyfluoroethylene, polyethyleneoxy, and polyacrylamide, as well as co-polymers and grafts thereof. A solid support can also be inorganic, such as glass, silica, controlled pore glass (CPG), reverse phase silica or metal, such as gold or platinum. A solid support can also consist of magnetic particles. For an overview of suitable support materials for enzyme immobilization see Zdarta et al. Catalysts 2018, 8, 92, and Datta et al. Biotech 2013 3:1-9.

The configuration of a solid support can be in the form of beads, monoliths, spheres, particles, a particle bed, a fiber mat, granules, a gel, a membrane, a hollow-fiber membrane, a mixed-matrix membrane or a surface. Surfaces can be planar, substantially planar, or non-planar. Solid supports can be porous or nonporous, and can have swelling or non-swelling characteristics. A solid support can be configured in the form of a well, depression, or other container, vessel, feature, or location.

As used herein, the term **"carbon source"** refers to a substrate or compound suitable for use as a source of carbon for the growth of the genetically engineered cell. Carbon sources can take a variety of forms including, but not limited to, polymers, carbohydrates, acids, alcohols, aldehydes, ketones, amino acids, peptides, and gases (e.g. CO and CO₂). Exemplary carbon sources include monosaccharides such as glucose, fructose, mannose, galactose, xylose and arabinose; oligosaccharides such as fructooligosaccharides and galactooligosaccharides; polysaccharides such as starch, cellulose, pectin and xylan; disaccharides such as Sucrose, maltose, cellobiose and turanose; cellulosic materials and variants such as hemicellulose, methylcellulose and sodium carboxymethylcellulose; succinates, lactates and acetates; alcohols such as ethanol, methanol and glycerol, or mixtures thereof, without limitation included. The carbon source may also be a product of photosynthesis, such as glucose. In some embodiments, the carbon source is biomass. In other embodiments, the carbon source is glucose. In other embodiments, the carbon source is sucrose.

The percentage of **"sequence identity"** is determined by comparing two optimally aligned nucleic acid or polypeptide sequences over a "comparison window" on the full length of the reference sequence. A **"comparison window"** as used herein, refers to the optimal alignment between the reference and variant sequence after that the two sequences are optimally aligned, wherein the variant nucleic acid or polypeptide sequence in the comparison window may comprise additions or deletions (i.e., gaps) of 20 percent or less, usually 5 to 15 percent, or 10 to 12 percent, as compared to the reference sequences (which does not comprise additions or deletions) for optimal alignment. Identity percentage is calculated by determining the number of positions at which the identical nucleic acid bases or amino acid residues occurs in both sequences to yield the number of matched positions, dividing the number of matched positions by the total number of positions in the reference sequence (i.e., the full length in amino acid or nucleotide) and multiplying the results by 100 to yield the percentage of sequence identity. Two nucleic acid or polypeptide sequences are said to be **"identical"** if the sequence of nucleotides or amino acids in the two sequences is the same when optimally aligned as described above.

Thus, the present invention is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide.

In a preferred embodiment, the enzyme with β-galactoside α-sialyltransferase activity consists of an amino acid sequence with at least 80% identity to SEQ ID NOs: 4-9, more preferably with at least 85% identity to SEQ ID NOs: 4-9, more preferably with at least 90% identity to SEQ ID NOs: 4-9, more preferably at least 92% identity to SEQ ID NOs: 4-9, more preferably with at least 93% identity to SEQ ID NOs: 4-9, more preferably with at least 94% identity to SEQ ID NOs: 4 - 9, more preferably with at least 95% identity to SEQ ID NOs: 4 - 9, more preferably with at least 96% identity to SEQ ID NOs: 4-9, more preferably with at least 97% identity to SEQ ID NOs: 4-9, more preferably with at least 98% identity to SEQ ID NOs: 4-9, and most preferably with at least 99% identity to SEQ ID NOs: 4-9.

In another preferred embodiment, the method for producing an α-sialyl-β-D-galactoside saccharide, comprises
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 5 or an amino acid sequence with at least 80% identity to SEQ ID NOs: 4 - 5;
B) mixing said solution and said enzyme and reacting a resulting solution to produce the α-sialyl-β-D-galactoside saccharide,
   wherein the α-sialyl-β-D-galactoside saccharide is an α-sialyl-(2→6)-β-D-galactoside saccharide and the enzyme with β-galactoside α-sialyltransferase activity is an enzyme with β-galactoside α-2,6-sialyltransferase activity consisting of an amino acid sequence as set forth in SEQ ID NOs: 4 - 5 or an amino acid sequence with at least 80% identity to SEQ ID NOs: 4 - 5

Reworded, the present invention is directed to a method for producing an α-sialyl-(2→6)-β-D-galactoside saccharide, comprising
A) providing a solution comprising a β-D-galactoside saccharide, a sialic acid donor, and
   an enzyme with β-galactoside α-2,6-sialyltransferase activity consisting of an amino acid sequence as set forth in SEQ ID NOs: 4 - 5 or an amino acid sequence with at least 80% identity to SEQ ID NOs: 4 - 5;
B) mixing said solution and said enzyme and reacting a resulting solution to produce the α-sialyl-(2→6)-β-D-galactoside saccharide.

In a preferred embodiment, the enzyme with β-galactoside α-2,6-sialyltransferase activity consists of an amino acid sequence with at least 80% identity to SEQ ID NOs: 4-5, more preferably with at least 85% identity to SEQ ID NOs: 4-5, more preferably with at least 90% identity to SEQ ID NOs: 4-5, more preferably at least 92% identity to SEQ ID NOs: 4-5, more preferably with at least 93% identity to SEQ ID NOs: 4-5, more preferably with at least 94% identity to SEQ ID NOs: 4 - 5, more preferably with at least 95% identity to SEQ ID NOs: 4 - 5, more preferably with at least 96% identity to SEQ ID NOs: 4-9, more preferably with at least 97% identity to SEQ ID NOs: 4-5, more preferably with at least 98% identity to SEQ ID NOs: 4 - 5, and most preferably with at least 99% identity to SEQ ID NOs: 4-5.

In another preferred embodiment, the method for producing an α-sialyl-β-D-galactoside saccharide, comprises
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the α-sialyl-β-D-galactoside saccharide is an α-sialyl-(2→3)-β-D-galactoside saccharide and the enzyme with β-galactoside α-sialyltransferase activity is an enzyme with β-galactoside α-2,3-sialyltransferase activity consisting of an amino acid sequence as set forth in SEQ ID NOs: 4 - 5 or an amino acid sequence with at least 80% identity to SEQ ID NOs: 4 - 5

Reworded, the present invention is directed to a method for producing an α-sialyl-(2→3)-β-D-galactoside saccharide, comprising
A) providing a solution comprising a β-D-galactoside saccharide, a sialic acid donor, and
   an enzyme with β-galactoside α-2,3-sialyltransferase activity consisting of an amino acid sequence as set forth in SEQ ID NOs: 6 - 9 or an amino acid sequence with at least 80% identity to SEQ ID NOs: 6 - 9;
B) mixing said solution and said enzyme and reacting a resulting solution to produce the α-sialyl-(2→3)-β-D-galactoside saccharide.

In a preferred embodiment, the enzyme with β-galactoside α-2,3-sialyltransferase activity consists of an amino acid sequence with at least 80% identity to SEQ ID NOs: 6-9, more preferably with at least 85% identity to SEQ ID NOs: 6-9, more preferably with at least 90% identity to SEQ ID NOs: 6-9, more preferably at least 92% identity to SEQ ID NOs: 6-9, more preferably with at least 93% identity to SEQ ID NOs: 6-9, more preferably with at least 94% identity to SEQ ID NOs: 6 - 9, more preferably with at least 95% identity to SEQ ID NOs: 6 - 9, more preferably with at least 96% identity to SEQ ID NOs: 6-9, more preferably with at least 97% identity to SEQ ID NOs: 6-9, more preferably with at least 98% identity to SEQ ID NOs: 6-9, and most preferably with at least 99% identity to SEQ ID NOs: 6-9.

Another preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the enzyme with β-galactoside α-sialyltransferase activity does not exhibit a hydrolase activity towards the sialic acid donor.

Another preferred embodiment is directed to a method for producing an α-sialyl-β-D-galactoside saccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4, 7, and 8 or an amino acid sequence with at least 80% identity to SEQ ID NOs: 4, 7, and 8;
B) mixing said solution and said enzyme and reacting a resulting solution to produce the α-sialyl-β-D-galactoside saccharide.

In a preferred embodiment, the enzyme with β-galactoside α-sialyltransferase activity consists of an amino acid sequence with at least 80% identity to SEQ ID NOs: 4, 7, and 8, more preferably with at least 85% identity to SEQ ID NOs: 4, 7, and 8, more preferably with at least 90% identity to SEQ ID NOs: 4, 7, and 8, more preferably at least 92% identity to SEQ ID NOs: 4, 7, and 8, more preferably with at least 93% identity to SEQ ID NOs: 4, 7, and 8, more preferably with at least 94% identity to SEQ ID NOs: 4, 7, and 8, more preferably with at least 95% identity to SEQ ID NOs: 4, 7, and 8, more preferably with at least 96% identity to SEQ ID NOs: 4, 7, and 8, more preferably with at least 97% identity to SEQ ID NOs: 4, 7, and 8, more preferably with at least 98% identity to SEQ ID NOs: 4, 7, and 8, and most preferably with at least 99% identity to SEQ ID NOs: 4, 7, and 8.

In a preferred embodiment, the β-D-galactoside saccharide is a glycopeptide, a glycoprotein, a glycolipid, a glycan or a human milk oligosaccharide (HMO).

Reworded, the present invention is directed to a method for producing an α-sialyl-β-D-galactoside glycopeptide, glycoprotein, glycolipid, glycan or human milk oligosaccharide, comprising
A) providing a solution comprising a β-D-galactoside glycopeptide, glycoprotein, glycolipid, glycan or human milk oligosaccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside glycopeptide, glycoprotein, glycolipid, glycan or human milk oligosaccharide.

Reworded, the present invention is directed to a method for producing an α-sialyl-(2→6)-β-D-galactoside glycopeptide, glycoprotein, glycolipid, glycan or human milk oligosaccharide, comprising
A) providing a solution comprising a β-D-galactoside glycopeptide, glycoprotein, glycolipid, glycan or human milk oligosaccharide, a sialic acid donor, and
   an enzyme with β-galactoside α-sialyltransferase activity consisting of an amino acid sequence as set forth in SEQ ID NOs: 4 - 5 or an amino acid sequence with at least 80% identity to SEQ ID NOs: 4 - 5;
B) mixing said solution and said enzyme and reacting a resulting solution to produce the α-sialyl-(2→6)-β-D-galactoside glycopeptide, glycoprotein, glycolipid, glycan or human milk oligosaccharide.

Reworded, the present invention is directed to a method for producing an α-sialyl-(2→3)-β-D-galactoside glycopeptide, glycoprotein, glycolipid, glycan or human milk oligosaccharide, comprising
A) providing a solution comprising a β-D-galactoside glycopeptide, glycoprotein, glycolipid, glycan or human milk oligosaccharide, a sialic acid donor, and
   an enzyme with β-galactoside α-sialyltransferase activity consisting of an amino acid sequence as set forth in SEQ ID NOs: 6 - 9 or an amino acid sequence with at least 80% identity to SEQ ID NOs: 6 - 9;
B) mixing said solution and said enzyme and reacting a resulting solution to produce the α-sialyl-(2→3)-β-D-galactoside glycopeptide, glycoprotein, glycolipid, glycan or human milk oligosaccharide.

In a preferred embodiment, the β-D-galactoside saccharide comprises one or more terminal β-D-galactose residues.

Reworded, the present invention is directed to a method for producing an α-sialyl-(2→6)-β-D-galactoside saccharide, comprising
A) providing a solution comprising a β-D-galactoside saccharide, a sialic acid donor, and
   an enzyme with β-galactoside α-2,6-sialyltransferase activity consisting of an amino acid sequence as set forth in SEQ ID NOs: 4 - 5 or an amino acid sequence with at least 80% identity to SEQ ID NOs: 4 - 5;
B) mixing said solution and said enzyme and reacting a resulting solution to produce the α-sialyl-(2→6)-β-D-galactoside saccharide,
   wherein the β-D-galactoside saccharide comprises one or more terminal β-D-galactose residues.

Reworded, the present invention is directed to a method for producing an α-sialyl-(2→3)-β-D-galactoside saccharide, comprising
A) providing a solution comprising a β-D-galactoside saccharide, a sialic acid donor, and
   an enzyme with β-galactoside α-2,3-sialyltransferase activity consisting of an amino acid sequence as set forth in SEQ ID NOs: 6 - 9 or an amino acid sequence with at least 80% identity to SEQ ID NOs: 6 - 9;
B) mixing said solution and said enzyme and reacting a resulting solution to produce the α-sialyl-(2→3)-β-D-galactoside saccharide,
   wherein the β-D-galactoside saccharide comprises one or more terminal β-D-galactose residues.

In a preferred embodiment, the α-sialyl-β-D-galactoside saccharide is a sialylated human milk oligosaccharide selected from 6'-siallylactose, 3'-siallylactose, sialyllacto-N-tetraose a (LSTa), sialyllacto-N-neotetraose d (LSTd), sialyllacto-N-neotetraose c (LSTc), sialyllacto-N-tetraose b (LSTb) and disialyllacto-N-tetraose (DSLNT).

Reworded, the present invention is directed to a method for producing an α-sialyl-β-D-galactoside human milk oligosaccharide, comprising
A) providing a solution comprising a β-D-galactoside saccharide, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside human milk oligosaccharide,
   wherein the α-sialyl-β-D-galactoside human milk oligosaccharide is selected from sialylated human milk oligosaccharide selected from 6'-siallylactose, 3'-siallylactose, sialyllacto-N-tetraose a (LSTa), sialyllacto-N-neotetraose d (LSTd), sialyllacto-N-neotetraose c (LSTc), sialyllacto-N-tetraose b (LSTb) and disialyllacto-N-tetraose (DSLNT).

Preferably, the α-sialyl-β-D-galactoside human milk oligosaccharide is 6'siallylactose.

Another embodiment is directed to a method for producing an α-sialyl-β-D-galactoside glycoprotein, comprising
A) providing a solution comprising a β-D-galactoside glycoprotein comprising A2G2 glycan, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside glycoprotein comprising A2G2S1 and/or A2G2S2.

Another embodiment is directed to a method for producing an α-sialyl-β-D-galactoside monoclonal antibody, comprising
A) providing a solution comprising a monoclonal antibody comprising a β-D-galactoside, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside monoclonal antibody.

Another embodiment is directed to a method for producing an α-(2→6)-sialyl-β-D-galactoside monoclonal antibody, comprising
A) providing a solution comprising a monoclonal antibody comprising a β-D-galactoside, a sialic acid donor, and
   an enzyme with β-galactoside α-2,6-sialyltransferase activity consisting of an amino acid sequence as set forth in SEQ ID NOs: 4 - 5 or an amino acid sequence with at least 80% identity to SEQ ID NOs: 4 - 5;
B) mixing said solution and said enzyme and reacting a resulting solution to produce α-(2→6)-sialyl-β-D-galactoside monoclonal antibody.

Another embodiment is directed to a method for producing an α-(2→3)-sialyl-β-D-galactoside monoclonal antibody, comprising
A) providing a solution comprising a monoclonal antibody comprising a β-D-galactoside, a sialic acid donor, and
   an enzyme with β-galactoside α-2,3-sialyltransferase activity consisting of an amino acid sequence as set forth in SEQ ID NOs: 6 - 9 or an amino acid sequence with at least 80% identity to SEQ ID NOs: 6 - 9;
B) mixing said solution and said enzyme and reacting a resulting solution to produce α-(2→3)-sialyl-β-D-galactoside monoclonal antibody.

Another embodiment is directed to a method for producing an α-sialyl-β-D-galactoside monoclonal antibody, comprising
A) providing a solution comprising a monoclonal antibody comprising a β-D-galactoside, a sialic acid donor, and
   an enzyme with β-galactoside α-sialyltransferase activity consisting of an amino acid sequence as set forth in SEQ ID NOs: 4, 7, and 8 or an amino acid sequence with at least 80% identity to SEQ ID NOs: 4, 7, and 8;
B) mixing said solution and said enzyme and reacting a resulting solution to produce α-sialyl-β-D-galactoside monoclonal antibody.

Preferably, monoclonal antibody is a bispecific monoclonal antibody or an antibody-based drug. Preferably, the monoclonal antibody is not fully sialylated. Preferably, the monoclonal antibody is a therapeutic protein is selected from the group consisting of:
3F8, 8H9, Arcitumomab, Ascrinvacumab, Aselizumab, Atezolizumab, Atidortoxumab, Atinuma, Atorolimumab, Avelumab, Azintuxizumab vedotin, Bapineuzumab, Basiliximab, Bavituximab, BCD-100, Bectumomab, Begelomab, Belantamab mafodotin, Belimumab, Bemarituzuma, Benralizumab, Berlimatoxumab, Bermekimab, Bersanlimab, Bertilimumab, Besilesomab, Bevacizumab, Bezlotoxumab, Biciromab, Bimagrumab, Bimekizumab, Birtamimab, Bivatuzumab mertansine, Bleselumab, Blinatumomab, Blontuvetmab, Blosozumab, Bococizumab, Brazikumab, Brentuximab vedotin, Briakinumab, Brodalumab, Brolucizumab, Brontictuzumab, Burosumab, Cabiralizumab, Camidanlumab tesirine, Camrelizumab, Canakinumab, Cantuzumab mertansine, Cantuzumab ravtansine, Caplacizumab, Capromab pendetide, Carlumab, Carotuximab, Catumaxomab, cBR96-doxorubicin immunoconjugate, Cedelizumab, Cemiplimab, Cergutuzumab amunaleukin, Certolizumab pegol, Cetrelimab, Cetuximab, Cibisatamab, Cirmtuzumab, Citatuzumab bogatox, Cixutumumab, Clazakizumab, Clenoliximab, Clivatuzumab tetraxetan, Codrituzumab, Cofetuzumab pelidotin, Coltuximab ravtansine, Conatumumab, Concizumab, Cosfroviximab, CR6261, Crenezumab, Crizanlizumab, Crotedumab, Cusatuzumab, Dacetuzumab, Daclizumab, Dalotuzumab, Dapirolizumab pegol, Daratumumab, Dectrekumab, Demcizumab, Denintuzumab mafodotin, Denosumab, Depatuxizumab mafodotin, Derlotuximab biotin, Detumomab, Dezamizumab, Dinutuximab, Diridavumab, Domagrozumab, Dorlimomab aritox, Dostarlima, Drozitumab, DS-8201, Duligotuzumab, Dupilumab, Durvalumab, Dusigitumab, Duvortuxizumab, Ecromeximab, Eculizumab, Edobacomab, Edrecolomab, Efalizumab, Efungumab, Eldelumab, Elezanumab, Elgemtumab, Elotuzumab, Elsilimomab, Emactuzumab, Emapalumab, Emibetuzumab, Emicizumab, Enapotamab vedotin, Enavatuzumab, Enfortumab vedotin, Enlimomab pegol, Enoblituzumab, Enokizumab, Enoticumab, Ensituximab, Epitumomab cituxetan, Epratuzumab, Eptinezumab, Erenumab, Erlizumab, Ertumaxomab, Etaracizumab, Etigilimab, Etrolizumab, Evinacumab, Evolocumab, Exbivirumab, Fanolesomab, Faralimomab, Faricimab, Farletuzumab, Fasinumab, FBTA05 , Felvizumab, Fezakinumab , Fibatuzumab, Ficlatuzumab, Figitumumab, Firivumab, Flanvotumab, Fletikumab, Flotetuzumab, Fontolizumab, Foralumab, Foravirumab, Fremanezumab, Fresolimumab, Frovocimab, Frunevetmab, Fulranumab, Futuximab, Galcanezumab, Galiximab, Gancotama, Ganitumab, Gantenerumab, Gatipotuzumab, Gavilimomab, Gedivumab, Gemtuzumab ozogamicin, Gevokizumab, Gilvetmab, Gimsilumab, Girentuximab, Glembatumumab vedotin, Golimumab, Gomiliximab, Gosuranemab, Guselkumab, Ianalumab, Ibalizumab, IBI308, Ibritumomab tiuxetan, Icrucumab, Idarucizumab, Ifabotuzumab, Igovomab, Iladatuzumab vedotin, IMAB362, Imalumab, Imaprelimab, Imciromab, Imgatuzumab, Inclacumab, Indatuximab ravtansine, Indusatumab vedotin, Inebilizumab, Infliximab, Inolimomab, Inotuzumab ozogamicin, Intetumumab , Iomab-B, Ipilimumab, Iratumumab, Isatuximab, Iscalimab, Istiratumab, Itolizumab, Ixekizumab, Keliximab, Labetuzumab, Lacnotuzumab, Ladiratuzumab vedotin, Lampalizumab, Lanadelumab, Landogrozumab, Laprituximab emtansine, Larcaviximab, Lebrikizumab, Lemalesomab, Lendalizumab, Lenvervimab, Lenzilumab, Lerdelimumab, Leronlimab, Lesofavumab, Letolizumab, Lexatumumab, Libivirumab, Lifastuzumab vedotin, Ligelizumab, Lilotomab satetraxetan, Lintuzumab, Lirilumab, Lodelcizumab, Lokivetmab, Loncastuximab tesirine, Lorvotuzumab mertansine, Losatuxizumab vedotin, Lucatumumab, Lulizumab pegol, Lumiliximab, Lumretuzumab, Lupartumab amadotin, Lutikizumab, Mapatumumab, Margetuximab, Marstacima, Maslimomab, Matuzumab, Mavrilimumab, Mepolizumab, Metelimumab, Milatuzumab, Minretumomab, Mirikizumab, Mirvetuximab soravtansine, Mitumomab, Modotuximab, Mogamulizumab, Monalizumab, Morolimumab, Mosunetuzumab, Motavizumab, Moxetumomab pasudotox, Muromonab-CD3, Nacolomab tafenatox, Namilumab, Naptumomab estafenatox, Naratuximab emtansine, Narnatumab, Natalizumab, Navicixizumab, Navivumab, Naxitamab, Nebacumab, Necitumumab, Nemolizumab, NEOD001, Nerelimomab, Nesvacumab, Netakimab, Nimotuzumab , Nirsevimab, Nivolumab, Nofetumomab merpentan, Obiltoxaximab, Obinutuzumab, Ocaratuzumab, Ocrelizumab, Odulimomab, Ofatumumab, Olaratumab, Oleclumab, Olendalizumab, Olokizumab, Omalizumab, Omburtamab, OMS721, Onartuzumab, Ontuxizumab, Onvatilimab, Opicinumab, Oportuzumab monatox, Oregovomab, Orticumab, Otelixizumab, Otilimab, Otlertuzumab, Oxelumab, Ozanezumab, Ozoralizumab, Pagibaximab, Palivizumab, Pamrevlumab, Panitumumab, Pankomab, Panobacumab, Parsatuzumab, Pascolizumab, Pasotuxizumab, Pateclizumab, Patritumab, PDR001, Pembrolizumab, Pemtumomab, Perakizumab, Pertuzumab, Pexelizumab, Pidilizumab, Pinatuzumab vedotin, Pintumomab, Placulumab, Plozalizumab, Pogalizumab, Polatuzumab vedotin, Ponezumab, Porgaviximab, Prasinezumab, Prezalizumab, Priliximab, Pritoxaximab, Pritumumab, PRO 140, Quilizumab, Racotumomab, Radretumab, Rafivirumab, Ralpancizumab, Ramucirumab, Ranevetmab, Ranibizumab, Ravagalimab, Ravulizumab, Raxibacumab, Refanezumab, Regavirumab, Relatlimab, Remtolumab, Reslizumab, Rilotumumab, Rinucumab, Risankizumab, Rituximab, Rivabazumab pegol, Rmab, Robatumumab, Roledumab, Romilkimab, Romosozumab, Rontalizumab, Rosmantuzumab, Rovalpituzumab tesirine, Rovelizumab, Rozanolixizumab, Ruplizumab, SA237, Sacituzumab govitecan, Samalizumab, Samrotamab vedotin, Sarilumab, Satralizumab, Satumomab pendetide, Secukinumab, Selicrelumab, Seribantumab, Setoxaximab, Setrusumab, Sevirumab, SGN-CD19A, SHP647, Sibrotuzumab, Sifalimumab, Siltuximab, Simtuzumab, Siplizumab, Sirtratumab vedotin, Sirukumab, Sofituzumab vedotin, Solanezumab, Solitomab, Sonepcizumab, Sontuzumab, Spartalizumab, Stamulumab , Sulesomab, Suptavumab, Sutimlimab, Suvizumab, Suvratoxumab, Tabalumab, Tacatuzumab tetraxetan, Tadocizumab, Talacotuzumab, Talizumab, Tamtuvetmab, Tanezumab, Taplitumomab paptox, Tarextumab, Tavolimab, Tefibazumab, Telimomab aritox, Telisotuzumab vedotin, Tenatumomab, Teneliximab, Teplizumab, Tepoditamab, Teprotumumab, Tesidolumab, Tetulomab, Tezepelumab, TGN1412, Tibulizumab, Tigatuzumab, Tildrakizumab, Timigutuzumab, Timolumab, Tiragotumab, Tislelizumab, Tisotumab vedotin, TNX-650, Tocilizumab, Tomuzotuximab, Toralizumab, Tosatoxumab, Tositumomab, Tovetumab, Tralokinumab, Trastuzumab, Trastuzumab emtansine, TRBS07, Tregalizumab, Tremelimumab, Trevogrumab, Tucotuzumab celmoleukin , Tuvirumab, Ublituximab, Ulocuplumab, Urelumab, Urtoxazumab, Ustekinumab, Utomilumab, Vadastuximab talirine, Vanalimab, Vandortuzumab vedotin, Vantictumab, Vanucizumab, Vapaliximab, Varisacumab, Varlilumab, Vatelizumab, Vedolizumab, Veltuzumab, Vepalimomab, Vesencumab, Visilizumab, Vobarilizumab, Volociximab, Vonlerolizumab, Vopratelimab, Vorsetuzumab mafodotin, Votumumab, Vunakizumab, Xentuzumab, XMAB-5574, Zalutumumab, Zanolimumab, Zatuximab, Zenocutuzumab, Ziralimumab, Zolbetuximab (=IMAB36, Claudiximab), and Zolimomab aritox.

In a preferred embodiment, the sialic acid donor is a sialic acid nucleotide, preferably CMP-Neu5Ac. Thus, the present invention is directed to a method for producing an α-sialyl-β-D-galactoside saccharide, comprising
A) providing a solution comprising a β-D-galactoside saccharide, CMP-Neu5Ac, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide.

A preferred embodiment is directed to a method for producing an α-sialyl--(2→6)-β-D-galactoside saccharide, comprising
A) providing a solution comprising a β-D-galactoside saccharide, CMP-Neu5Ac, and
   an enzyme with β-galactoside α-2,6-sialyltransferase activity consisting of an amino acid sequence as set forth in SEQ ID NOs: 4 - 5 or an amino acid sequence with at least 80% identity to SEQ ID NOs: 4 - 5;
B) mixing said solution and said enzyme and reacting a resulting solution to produce the α--(2→6)-sialyl-β-D-galactoside saccharide.

A preferred embodiment is directed to a method for producing an α-sialyl--(2→3)-β-D-galactoside saccharide, comprising
A) providing a solution comprising a β-D-galactoside saccharide, CMP-Neu5Ac, and
   an enzyme with β-galactoside α-2,3-sialyltransferase activity consisting of an amino acid sequence as set forth in SEQ ID NOs: 6 - 9 or an amino acid sequence with at least 80% identity to SEQ ID NOs: 6 - 9;
B) mixing said solution and said enzyme and reacting a resulting solution to produce the α--(2→3)-sialyl-β-D-galactoside saccharide.

A preferred embodiment is directed to a method for producing an α-sialyl-β-D-galactoside saccharide, comprising
A) providing a solution comprising a β-D-galactoside saccharide, CMP-Neu5Ac, and
   an enzyme with β-galactoside α-sialyltransferase activity consisting of an amino acid sequence as set forth in SEQ ID NOs: 4, 7, and 8 or an amino acid sequence with at least 80% identity to SEQ ID NOs: 4, 7, and 8;
B) mixing said solution and said enzyme and reacting a resulting solution to produce the α-sialyl-β-D-galactoside saccharide.

A preferred embodiment is directed to a method for producing an α-sialyl-β-D-galactoside glycopeptide, glycoprotein, glycolipid, glycan or human milk oligosaccharide, comprising
A) providing a solution comprising a β-D-galactoside glycopeptide, glycoprotein, glycolipid, glycan or human milk oligosaccharide, CMP-Neu5Ac, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside glycopeptide, glycoprotein, glycolipid, glycan or human milk oligosaccharide.

Reworded, the present invention is directed to a method for producing an α-sialyl-(2→6)-β-D-galactoside glycopeptide, glycoprotein, glycolipid, glycan or human milk oligosaccharide, comprising
A) providing a solution comprising a β-D-galactoside glycopeptide, glycoprotein, glycolipid, glycan or human milk oligosaccharide, CMP-Neu5Ac, and
   an enzyme with β-galactoside α-sialyltransferase activity consisting of an amino acid sequence as set forth in SEQ ID NOs: 4 - 5 or an amino acid sequence with at least 80% identity to SEQ ID NOs: 4 - 5;
B) mixing said solution and said enzyme and reacting a resulting solution to produce the α-sialyl-(2→6)-β-D-galactoside glycopeptide, glycoprotein, glycolipid, glycan or human milk oligosaccharide.

Reworded, the present invention is directed to a method for producing an α-sialyl-(2→3)-β-D-galactoside glycopeptide, glycoprotein, glycolipid, glycan or human milk oligosaccharide, comprising
A) providing a solution comprising a β-D-galactoside glycopeptide, glycoprotein, glycolipid, glycan or human milk oligosaccharide, CMP-Neu5Ac, and
   an enzyme with β-galactoside α-sialyltransferase activity consisting of an amino acid sequence as set forth in SEQ ID NOs: 6 - 9 or an amino acid sequence with at least 80% identity to SEQ ID NOs: 6 - 9;
B) mixing said solution and said enzyme and reacting a resulting solution to produce the α-sialyl-(2→3)-β-D-galactoside glycopeptide, glycoprotein, glycolipid, glycan or human milk oligosaccharide.

In another embodiment, the present invention is directed to a method for producing an α-sialyl-(2→6)-β-D-galactoside saccharide, comprising
A) providing a solution comprising a β-D-galactoside saccharide, CMP-Neu5Ac, and
   an enzyme with β-galactoside α-2,6-sialyltransferase activity consisting of an amino acid sequence as set forth in SEQ ID NOs: 4 - 5 or an amino acid sequence with at least 80% identity to SEQ ID NOs: 4 - 5;
B) mixing said solution and said enzyme and reacting a resulting solution to produce the α-sialyl-(2→6)-β-D-galactoside saccharide,
   wherein the β-D-galactoside saccharide comprises one or more terminal β-D-galactose residues.

In another embodiment, the present invention is directed to a method for producing an α-sialyl-(2→3)-β-D-galactoside saccharide, comprising
A) providing a solution comprising a β-D-galactoside saccharide, CMP-Neu5Ac, and
   an enzyme with β-galactoside α-2,3-sialyltransferase activity consisting of an amino acid sequence as set forth in SEQ ID NOs: 6 - 9 or an amino acid sequence with at least 80% identity to SEQ ID NOs: 6 - 9;
B) mixing said solution and said enzyme and reacting a resulting solution to produce the α-sialyl-(2→3)-β-D-galactoside saccharide,
   wherein the β-D-galactoside saccharide comprises one or more terminal β-D-galactose residues.

In a preferred embodiment, the present invention is directed to a method for producing an α-sialyl-β-D-galactoside human milk oligosaccharide, comprising
A) providing a solution comprising a β-D-galactoside saccharide, CMP-Neu5Ac, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside human milk oligosaccharide,
   wherein the α-sialyl-β-D-galactoside human milk oligosaccharide is selected from sialylated human milk oligosaccharide selected from 6'-siallylactose, 3'-siallylactose, sialyllacto-N-tetraose a (LSTa), sialyllacto-N-neotetraose d (LSTd), sialyllacto-N-neotetraose c (LSTc), sialyllacto-N-tetraose b (LSTb) and disialyllacto-N-tetraose (DSLNT).

Preferably, the α-sialyl-β-D-galactoside human milk oligosaccharide is 6'-iallylactose.

Another embodiment is directed to a method for producing an α-sialyl-β-D-galactoside glycoprotein, comprising
A) providing a solution comprising a β-D-galactoside glycoprotein comprising A2G2 glycan, CMP-Neu5Ac, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside glycoprotein comprising A2G2S1 and/or A2G2S2.

Another embodiment is directed to a method for producing an α-sialyl-β-D-galactoside monoclonal antibody, comprising
A) providing a solution comprising a monoclonal antibody comprising a β-D-galactoside, CMP-Neu5Ac, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside monoclonal antibody.

Another embodiment is directed to a method for producing an α-(2→6)-sialyl-β-D-galactoside monoclonal antibody, comprising
A) providing a solution comprising a monoclonal antibody comprising a β-D-galactoside, CMP-Neu5Ac, and
   an enzyme with β-galactoside α-2,6-sialyltransferase activity consisting of an amino acid sequence as set forth in SEQ ID NOs: 4 - 5 or an amino acid sequence with at least 80% identity to SEQ ID NOs: 4 - 5;
B) mixing said solution and said enzyme and reacting a resulting solution to produce α-(2→6)-sialyl-β-D-galactoside monoclonal antibody.

Another embodiment is directed to a method for producing an α-(2→3)-sialyl-β-D-galactoside monoclonal antibody, comprising
A) providing a solution comprising a monoclonal antibody comprising a β-D-galactoside, CMP-Neu5Ac, and
   an enzyme with β-galactoside α-2,3-sialyltransferase activity consisting of an amino acid sequence as set forth in SEQ ID NOs: 6 - 9 or an amino acid sequence with at least 80% identity to SEQ ID NOs: 6 - 9;
B) mixing said solution and said enzyme and reacting a resulting solution to
   produce α-(2→3)-sialyl-β-D-galactoside monoclonal antibody.

Another embodiment is directed to a method for producing an α-sialyl-β-D-galactoside monoclonal antibody, comprising
A) providing a solution comprising a monoclonal antibody comprising a β-D-galactoside, CMP-Neu5Ac, and
   an enzyme with β-galactoside α-sialyltransferase activity consisting of an amino acid sequence as set forth in SEQ ID NOs: 4, 7, and 8 or an amino acid sequence with at least 80% identity to SEQ ID NOs: 4, 7, and 8;
B) mixing said solution and said enzyme and reacting a resulting solution to produce α-sialyl-β-D-galactoside monoclonal antibody.

Preferably, monoclonal antibody is a bispecific monoclonal antibody or an antibody-based drug. Preferably, the monoclonal antibody is not fully sialylated.

In some embodiments, the resulting solution in the step B) has a pH value in a range of 5.0 - 10.0, more preferred 5.5 - 9.5, and most preferred 6.0 - 9.0.

Preferably, the resulting solution is a buffer solution having a pH value in a range of 5.0 - 10.0, more preferred 5.5 - 9.5, and most preferred 6.0 - 9.0.

The buffer solution comprises at least one of acids and at least one of bases. Preferred the at least one of sulfonic acids is selected from the group consisting of citric acid, [tris(hydroxymethyl)methylamino]propanesulfonic acid (TAPS), 2-(bis(2-hydroxyethyl)amino)acetic acid (Bicine), tris(hydroxymethyl)aminomethane (Tris), N-[tris(hydroxymethyl)methyl]glycine (Tricine), 3-[N-tris(hydroxymethyl)-methylamino]-2-hydroxypropanesulfonic acid (TAPSO), 4-(2-hydroxyethyl)-1-piperazineethanesulfonic acid (HEPES), 2-[[1,3-dihydroxy-2-(hydroxymethyl)-propan-2-yl]amino]ethanesulfonic acid (TES), 3-(N-morpholino)propanesulfonic acid (MOPS), piperazine-N,N'-bis(2-ethanesulfonic acid (PIPES), 2-(N-morpholino)ethanesulfonic acid (MES).

Preferred the at least one of bases is selected from the group consisting of metal hydroxide, metal carbonate, metal bicarbonate, metal phosphate, metal biphosphate; more preferred, sodium hydroxide, calcium hydroxide, potassium hydroxide, sodium bicarbonate, sodium carbonate, calcium carbonate, potassium carbonate, monosodium phosphate, monocalcium phosphate, monopotassium phosphate, monomagnesium phosphate, disodium phosphate, calcium phosphate, and potassium phosphate.

Preferably, the buffer is ammonium bicarbonate.

Another embodiment is directed to a method for producing an α-sialyl-β-D-galactoside saccharide, comprising
A) providing a solution comprising a β-D-galactoside, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside saccharide,
   wherein the solution has a pH between 6 and 9.

Another embodiment is directed to a method for producing an α-sialyl-β-D-galactoside saccharide, comprising
A) providing a solution comprising a β-D-galactoside, CMP-Neu5Ac, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside saccharide,
   wherein the solution has a pH between 6 and 9.

Another embodiment is directed to a method for producing an α-sialyl-β-D-galactoside saccharide, comprising
A) providing a solution comprising a β-D-galactoside, a sialic acid donor, ammonium carbonate buffer, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside saccharide,
   wherein the solution has a pH between 6 and 9.

Another embodiment is directed to a method for producing an α-sialyl-β-D-galactoside saccharide, comprising
A) providing a solution comprising a β-D-galactoside, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside saccharide,
   wherein step B) is carried out at a reaction temperature between 25 °C and 50 °C.

Another embodiment is directed to a method for producing an α-sialyl-β-D-galactoside saccharide, comprising
A) providing a solution comprising a β-D-galactoside, CMP-Neu5Ac, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside saccharide,
   wherein step B) is carried out at a reaction temperature between 25 °C and 50 °C.

Another embodiment is directed to a method for producing an α-sialyl-β-D-galactoside saccharide, comprising
A) providing a solution comprising a β-D-galactoside, a sialic acid donor, and
   an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside saccharide,
   wherein step B) is carried out at a reaction temperature between 25 °C and 50 °C and wherein the solution has a pH between 6 and 9.

### In vitro preparation of α-sialyl-β-D-galactoside saccharides

Another aspect of the present invention is directed to an *in vitro* method for producing an α-sialyl-β-D-galactoside saccharide. To this extent, the method is performed cell free using isolated an enzyme with β-galactoside α-sialyltransferase activity.

Thus, the present invention is directed to an *in vitro* method for producing an α-sialyl-β-D-galactoside saccharide, comprising
A) providing a solution comprising a β-D-galactoside saccharide, a sialic acid donor, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide.

In a preferred embodiment, the isolated enzyme with β-galactoside α-sialyltransferase activity consists of an amino acid sequence with at least 80% identity to SEQ ID NOs: 4-9, more preferably with at least 85% identity to SEQ ID NOs: 4-9, more preferably with at least 90% identity to SEQ ID NOs: 4-9, more preferably at least 92% identity to SEQ ID NOs: 4-9, more preferably with at least 93% identity to SEQ ID NOs: 4-9, more preferably with at least 94% identity to SEQ ID NOs: 4-9, more preferably with at least 95% identity to SEQ ID NOs: 4-9, more preferably with at least 96% identity to SEQ ID NOs: 4-9, more preferably with at least 97% identity to SEQ ID NOs: 4-9, more preferably with at least 98% identity to SEQ ID NOs: 4-9, and most preferably with at least 99% identity to SEQ ID NOs: 4-9.

In another preferred embodiment, the *in vitro* method for producing an α-sialyl-β-D-galactoside saccharide, comprises
A) providing a solution comprising a β-D-galactoside saccharide, a sialic acid donor, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide.

In another preferred embodiment, the *in vitro* method for producing an α-(2→6)-sialyl-β-D-galactoside saccharide, comprises
A) providing a solution comprising a β-D-galactoside saccharide, a sialic acid donor, and
   an isolated enzyme with β-galactoside α-2,6-sialyltransferase activity consisting of an amino acid sequence as set forth in SEQ ID NOs: 4 - 5 or an amino acid sequence with at least 80% identity to SEQ ID NOs: 4 - 5;
B) mixing said solution and said enzyme and reacting a resulting solution to produce the α-(2→6)-sialyl-β-D-galactoside saccharide.

In another preferred embodiment, the *in vitro* method for producing an α-(2→3)-sialyl-β-D-galactoside saccharide, comprises
A) providing a solution comprising a β-D-galactoside saccharide, a sialic acid donor, and
   an isolated enzyme with β-galactoside α-2,3-sialyltransferase activity consisting of an amino acid sequence as set forth in SEQ ID NOs: 6 - 9 or an amino acid sequence with at least 80% identity to SEQ ID NOs: 6 - 9;
B) mixing said solution and said enzyme and reacting a resulting solution to produce the α-(2→3)-sialyl-β-D-galactoside saccharide.

In another preferred embodiment, the *in vitro* method for producing an α-sialyl-β-D-galactoside saccharide, comprises
A) providing a solution comprising a β-D-galactoside saccharide, a sialic acid donor, and
   an isolated enzyme with β-galactoside α-sialyltransferase activity consisting of an amino acid sequence as set forth in SEQ ID NOs: 4, 7, and 8 or an amino acid sequence with at least 80% identity to SEQ ID NOs: 4, 7, and 8;
B) mixing said solution and said enzyme and reacting a resulting solution to produce the α-sialyl-β-D-galactoside saccharide.

In a preferred embodiment, the β-D-galactoside saccharide is a glycopeptide, a glycoprotein, a glycolipid, a glycan or a human milk oligosaccharide (HMO).

Reworded, the present invention is directed to an *in vitro* method for producing an α-sialyl-β-D-galactoside glycopeptide, glycoprotein, glycolipid, glycan or human milk oligosaccharide, comprising
A) providing a solution comprising a β-D-galactoside glycopeptide, glycoprotein, glycolipid, glycan or human milk oligosaccharide, a sialic acid donor, and an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside glycopeptide, glycoprotein, glycolipid, glycan or human milk oligosaccharide.

Preferably, the *in vitro* method for producing an α-sialyl-β-D-galactoside glycopeptide, glycoprotein, glycolipid, glycan or human milk oligosaccharide, comprises
A) providing a solution comprising a β-D-galactoside glycopeptide, glycoprotein, glycolipid, glycan or human milk oligosaccharide, a sialic acid donor, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside glycopeptide, glycoprotein, glycolipid, glycan or human milk oligosaccharide.

In a preferred embodiment, the β-D-galactoside saccharide comprises one or more terminal β-D-galactose residues.

Reworded, the present invention is directed to an *in vitro* method for producing an α-sialyl-β-D-galactoside saccharide, comprising
A) providing a solution comprising a β-D-galactoside saccharide, a sialic acid donor, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the β-D-galactoside saccharide comprises one or more terminal β-D-galactose residues

In a preferred embodiment, the α-sialyl-(2→6)-β-D-galactoside saccharide is a sialylated human milk oligosaccharide selected from 6'-siallylactose, 3'-siallylactose, sialyllacto-N-tetraose a (LSTa), sialyllacto-N-neotetraose d (LSTd), sialyllacto-N-neotetraose c (LSTc), sialyllacto-N-tetraose b (LSTb) and disialyllacto-N-tetraose (DSLNT).

Reworded, the present invention is directed to an *in vitro* method for producing an α-sialyl-β-D-galactoside human milk oligosaccharide, comprising
A) providing a solution comprising a β-D-galactoside saccharide, a sialic acid donor, and an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside human milk oligosaccharide,
   wherein the α-sialyl-β-D-galactoside human milk oligosaccharide is selected from 6'-siallylactose, 3'-siallylactose, sialyllacto-N-tetraose a (LSTa), sialyllacto-N-neotetraose d (LSTd), sialyllacto-N-neotetraose c (LSTc), sialyllacto-N-tetraose b (LSTb) and disialyllacto-N-tetraose (DSLNT).

Preferably, the α-sialyl-β-D-galactoside human milk oligosaccharide is 6'-siallylactose.

Another embodiment is directed to an *in vitro* method for producing an α-sialyl-β-D-galactoside glycoprotein, comprising
A) providing a solution comprising a β-D-galactoside glycoprotein comprising A2G2 glycan, a sialic acid donor, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside glycoprotein comprising A2G2S1 and/or A2G2S2.

Another embodiment is directed to an *in vitro* method for producing an α-sialyl-β-D-galactoside monoclonal antibody, comprising
A) providing a solution comprising a monoclonal antibody comprising a β-D-galactoside, a sialic acid donor, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside monoclonal antibody.

In a preferred embodiment, the sialic acid donor is a sialic acid nucleotide, preferably CMP-Neu5Ac. Thus, the present invention is directed to an *in vitro* method for producing an α-sialyl-β-D-galactoside saccharide, comprising
A) providing a solution comprising a β-D-galactoside saccharide, CMP-Neu5Ac, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide.

In another preferred embodiment, the *in vitro* method for producing an a-sialyl-β-D-galactoside saccharide, comprises
A) providing a solution comprising a β-D-galactoside saccharide, CMP-Neu5Ac, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide.

In a preferred embodiment, the *in vitro* method for producing an α-sialyl-β-D-galactoside glycopeptide, glycoprotein, glycolipid, glycan or human milk oligosaccharide, comprises
A) providing a solution comprising a β-D-galactoside glycopeptide, glycoprotein, glycolipid, glycan or human milk oligosaccharide, CMP-Neu5Ac, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside glycopeptide, glycoprotein, glycolipid, glycan or human milk oligosaccharide.

Preferably, the in vitro method for producing an α-sialyl-β-D-galactoside glycopeptide, glycoprotein, glycolipid, glycan or human milk oligosaccharide, comprising
A) providing a solution comprising a β-D-galactoside glycopeptide, glycoprotein, glycolipid, glycan or human milk oligosaccharide, CMP-Neu5Ac, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside glycopeptide, glycoprotein, glycolipid, glycan or human milk oligosaccharide.

In a preferred embodiment, the *in vitro* method for producing an α-sialyl-β**-**D-galactoside saccharide, comprises
A) providing a solution comprising a β-D-galactoside saccharide, CMP-Neu5Ac, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide,
   wherein the β-D-galactoside saccharide comprises one or more terminal β-D-galactose residues

In a preferred embodiment, the *in vitro* method for producing an α-sialyl-β-D-galactoside human milk oligosaccharide, comprises
A) providing a solution comprising a β-D-galactoside saccharide, CMP-Neu5Ac, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside human milk oligosaccharide,
   wherein the α-sialyl-(2→6)-β-D-galactoside human milk oligosaccharide is selected from 6'-siallylactose, 3'-siallylactose, sialyllacto-N-tetraose a (LSTa), sialyllacto-N-neotetraose d (LSTd), sialyllacto-N-neotetraose c (LSTc), sialyllacto-N-tetraose b (LSTb) and disialyllacto-N-tetraose (DSLNT).

Preferably, the α-sialyl-β-D-galactoside human milk oligosaccharide is 6'-siallylactose.

Another embodiment is directed to an *in vitro* method for producing an α-sialyl-β-D-galactoside glycoprotein, comprising
A) providing a solution comprising a β-D-galactoside glycoprotein comprising A2G2 glycan, CMP-Neu5Ac, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside glycoprotein comprising A2G2S1 and/or A2G2S2.

Another embodiment is directed to an *in vitro* method for producing an α-sialyl-β-D-galactoside monoclonal antibody, comprising
A) providing a solution comprising a monoclonal antibody comprising a β-D-galactoside, CMP-Neu5Ac, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside monoclonal antibody.

In some embodiments, the resulting solution in the step B) has a pH value in a range of 5.0 - 10.0, more preferred 5.5 - 9.5, and most preferred 6.0 - 9.0.

Preferably, the resulting solution is a buffer solution having a pH value in a range of 5.0 - 10.0, more preferred 5.5 - 9.5, and most preferred 6.0 - 9.0.

Preferably, the buffer is an ammonium bicarbonate solution having a pH value in a range of 6.0 - 9.0.

Another embodiment is directed to an *in vitro* method for producing an α-sialyl-β-D-galactoside saccharide, comprising
A) providing a solution comprising a β-D-galactoside, a sialic acid donor, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside saccharide,
   wherein the solution has a pH between 6 and 9.

Another embodiment is directed to an *in vitro* method for producing an α-sialyl-β-D-galactoside saccharide, comprising
A) providing a solution comprising a β-D-galactoside, CMP-Neu5Ac, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside saccharide,
   wherein the solution has a pH between 6 and 9.

Another embodiment is directed to an *in vitro* method for producing an α-sialyl-β-D-galactoside saccharide, comprising
A) providing a solution comprising a β-D-galactoside, a sialic acid donor, ammonium carbonate buffer, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside saccharide,
   wherein the solution has a pH between 6 and 9.

Another embodiment is directed to an *in vitro* method for producing an α-sialyl-β-D-galactoside saccharide, comprising
A) providing a solution comprising a β-D-galactoside, a sialic acid donor, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside saccharide,
   wherein step B) is carried out at a reaction temperature between 25 °C and 50 °C.

Another embodiment is directed to an *in vitro* method for producing an α-sialyl-β-D-galactoside saccharide, comprising
A) providing a solution comprising a β-D-galactoside, CMP-Neu5Ac, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside saccharide,
   wherein step B) is carried out at a reaction temperature between 25 °C and 50 °C.

Another embodiment is directed to an *in vitro* method for producing an α-sialyl-β-D-galactoside saccharide, comprising
A) providing a solution comprising a β-D-galactoside, a sialic acid donor, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside saccharide,
   wherein step B) is carried out at a reaction temperature between 25 °C and 50 °C and wherein the solution has a pH between 6 and 9.

In a preferred embodiment, the isolated enzyme with β-galactoside α-sialyltransferase activity is immobilized on a solid support as disclosed herein. Thus, another embodiment is directed to an *in vitro* method for producing an α-sialyl-β-D-galactoside saccharide, comprising
A) providing a solution comprising a β-D-galactoside, a sialic acid donor, and
   an isolated enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce α-sialyl-β-D-galactoside saccharide,
   wherein the isolated enzyme is immobilized on a solid support, preferably a reusable, mechanically stable solid support.

### In vitro preparation of α-sialyl-β-D-galactoside saccharides using multi-enzyme cascade.

Another aspect of the present invention is directed to an *in vitro* method for producing an α-sialyl-β-D-galactoside saccharide, wherein the costly sialic acid donor CMP-Neu5Ac is formed *in situ* via a multienzyme cascade.

Thus, the present invention is also directed to an *in vitro* method for producing an α-sialyl---β-D-galactoside saccharide, comprising
A) providing a solution comprising a β-D-galactoside saccharide, a sialic acid donor, *N*-acetyl-D-glucosamine, pyruvate, polyphosphate, catalytic amounts of i) cytidine 5'-monophosphate, ii) cytidine 5'-diphosphate or iii) cytidine 5'-triphosphate, and catalytic amounts of i) adenosine 5'-monophosphate, adenosine 5'-diphosphate, or adenosine 5'-triphosphate, and
   a set of enzymes comprising an N-acylglucosamine 2-epimerase (AGE, EC 5.1.3.8), an N-acetylneuraminate lyase (NAL, EC 4.1.3.3), an N-acetylneuraminate cytidylyltransferase (CSS, EC 2.7.7.43), optional a uridine kinase (UDK, EC 2.7.1.48), a uridine monophosphate kinase (URA6, EC 2.7.4.22), a polyphosphate kinase 3 (PPK3, EC2.7.4.1), and an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide.

Reworded, the present invention is also directed to an *in vitro* method for producing an α-sialyl-β-D-galactoside saccharide, comprising
A) providing a solution comprising a β-D-galactoside saccharide, a sialic acid donor, *N*-acetyl-D-glucosamine, pyruvate, polyphosphate, catalytic amounts of i) cytidine 5'-monophosphate, ii) cytidine 5'-diphosphate or iii) cytidine 5'-triphosphate, and catalytic amounts of i) adenosine 5'-monophosphate, adenosine 5'-diphosphate, or adenosine 5'-triphosphate, and
   a set of enzymes comprising an N-acylglucosamine 2-epimerase (AGE, EC 5.1.3.8), an N-acetylneuraminate lyase (NAL, EC 4.1.3.3), an N-acetylneuraminate cytidylyltransferase (CSS, EC 2.7.7.43), optional a uridine kinase (UDK, EC 2.7.1.48), a uridine monophosphate kinase (URA6, EC 2.7.4.22), a polyphosphate kinase 3 (PPK3, EC2.7.4.1), and an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B1) mixing said solution and said enzyme;
B2) reacting a resulting solution to produce cytidine 5'-monophospho-*N*-acetyl-neuraminic acid (CMP-Neu5Ac); and
B3) reacting the CMP-Neu5Ac obtained after step B2) with β-D-galactoside saccharide to produce the α-sialyl-β-D-galactoside saccharide.

In a preferred embodiment the set of enzymes is co-immobilized on a solid support as disclosed herein.

Thus, the present invention is also directed to an *in vitro* method for producing an α-sialyl-β-D-galactoside saccharide, comprising
A) providing a solution comprising a β-D-galactoside saccharide, a sialic acid donor, *N*-acetyl-D-glucosamine, pyruvate, polyphosphate, catalytic amounts of i) cytidine 5'-monophosphate, ii) cytidine 5'-diphosphate or iii) cytidine 5'-triphosphate, and catalytic amounts of i) adenosine 5'-monophosphate, adenosine 5'-diphosphate, or adenosine 5'-triphosphate, and
   a set of enzymes comprising an N-acylglucosamine 2-epimerase (AGE, EC 5.1.3.8), an N-acetylneuraminate lyase (NAL, EC 4.1.3.3), an N-acetylneuraminate cytidylyltransferase (CSS, EC 2.7.7.43), optional a uridine kinase (UDK, EC 2.7.1.48), a uridine monophosphate kinase (URA6, EC 2.7.4.22), a polyphosphate kinase 3 (PPK3, EC2.7.4.1), and an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said set of enzymes and reacting a resulting solution to produce the α-sialyl-β-D-galactoside saccharide,
   wherein the set of enzymes is co-immobilized on a solid support.

The enzymes are immobilized on a solid support such that they retain their activity, substrate specificity, stereoselectivity and/or other properties. Suitable solid supports are for instance beads, monoliths, spheres, particles, a particle bed, a fiber mat, granules, a gel, a membrane, a hollow-fiber membrane, a mixed-matrix membrane, a surface or other solid phase material.

Surprisingly it has been found that co-immobilization of the set of enzymes results in a higher productivity in the production of α-sialyl-β-D-galactoside saccharides as well as cytidine 5'-monophospho-*N*-acetyl-neuraminic acid (CMP-Neu5Ac) compared to non-immobilized or separately immobilization of the enzymes. Thus, preferably the enzymes used in the inventive methods described herein are co-immobilized on a solid support.

Methods of enzyme immobilization are well-known in the art. The enzymes can be bound non-covalently or covalently, such as adsorption, covalent binding, ionic binding, metal binding, crosslinking or crystallization. Various methods for conjugation and immobilization of enzymes to solid supports (e.g., resins, membranes, beads, glass, etc.) are well known in the art and described in e.g.,: Yi et al., Process Biochemistry 2007, 42, 895; Martin et al., Applied Microbiology and Biotechnology 2007, 76, 843; Koszelewski et al., Journal of Molecular Catalysis B: Enzymatic, 2010, 63, 39; Truppo et al., Org. Process Res. Dev., 2011, 15, 1033; Hermanson, G.T., Bioconjugate Techniques, Second Edition, Academic Press (2008); Mateo et al., Biotechnology Progress, 2002, 18, 629; and Bioconjugation Protocols: Strategies and Methods, In Methods in Molecular Biology, C.M. Niemeyer ed., Humana Press (2004).

The enzymes used in the inventive methods described herein, namely an N-acylglucosamine 2-epimerase (AGE), an N-acetylneuraminate lyase (NAL), an N-acylneuraminate cytidylyltransferase (CSS), optional a uridine kinase (UDK), a uridine monophosphate kinase and a polyphosphate kinase 3 (PPK3), the inorganic diphosphatase (PPA), 1-domain polyphosphate kinase 2 (1DPPK2), 2-domain polyphosphate kinase 2 (2DPPK2), and pyrophosphatase are well known to the skilled person and can be obtained by any method well known to the skilled person in the art.

Particularly, the enzymes can be overexpressed in, isolated from or prepared by recombinant methods from microbiological cultures comprising bacterial cultures, such as *E*. *coli,* virus and phage cultures and eukaryotic cell cultures. The inventive methods described herein are not restricted to enzymes from the sources described in the experimental section. Thus, the inventive method can be performed with the above listed enzymes obtained from various sources using common protein expression or isolation techniques. Further, it is well known to the skilled person to adapt the preparation of the enzymes to the specific applications in which the method is used. For instance, the above listed enzymes can be expressed in E. coli by using bacterial growth media of non-animal origin, such as a Luria-Bertani broth comprising tryptone from soy.

The enzyme-containing solutions obtained from cell homogenization or cell lysis, which are usually centrifuged and filtered to remove cell debris, can be directly used for immobilizing the enzymes on a solid support. Thus, no further purification step or isolation step is required and the crude cell lysate or cell homogenate can be used for immobilizing the enzymes on a solid support such that they retain their activity, substrate specificity, stereoselectivity and/or other properties.

**Solid supports** useful for immobilizing the enzymes used in the method of the present invention include but are not limited to beads, monoliths, spheres, particles, a particle bed, a fiber mat, granules, a gel, a membrane, a hollow-fiber membrane, a mixed-matrix membrane or a surface. Preferably, the solid support has the form of beads.

In particular, the solid support is composed of beads or resins comprising a polymer with epoxide functional groups, with amino epoxide functional groups, with ethylenediamine functional groups, with amino C2 functional groups, with amino C6 functional groups, with anionic/amino C6 spacer functional groups. Preferably, the solid support is composed of porous beads having a pore size of 0.1 Å to 100000 Å.

Particularly preferred are solid supports that are functionalized with epoxide functional groups. Further preferred solid supports include, but are not limited to solid supports with ethylenediamine functional groups, with epoxy functional groups and further functionalized with a hydrophobic group, such as butyl, octyl, methyl, phenyl, for example with epoxide functional groups and butyl functional groups, with amino C2 spacer functional groups, with amino C6 spacer functional groups, or other amino spacer such as amino C3 spacer, amino C4 spacer, amino C5 spacer, amino C7 spacer, with epoxy functional groups, with anionic/amino C6 spacer functional groups, with anionic/tertiary amine functional groups, anionic/quaternary amine functional groups, with cationic/sulphonic functional groups, with carboxylic ester functional groups, with phenyl functional groups, with octadecyl functional groups, with styrene/methyl functional groups, macroporous resins or beads. The solid support may consist of a polymeric material, non-polymeric material, e.g. silica gel. The solid support may consists of a polymeric material including, but not limited to polymethacrylate, polyacrylic acid, acrylic polymer, polystyrene, styrene, styrene /methacrylate and mixtures thereof..

Examples of solid supports useful for immobilizing the enzymes used in the method of the present invention include but are not limited to beads or resins comprising polymethacrylate with epoxide functional groups, , polymethacrylate with amino epoxide functional groups, polymethacrylate with ethylenediamine functional groups, polymethacrylate with epoxide functional groups and further functionalized with a hydrophobic group, such as butyl, octyl, methyl, phenyl, for example polymethacrylate with epoxide functional groups and butyl functional groups, polymethacrylate with amino C2 spacer functional groups, polymethacrylate with amino C6 spacer functional groups, polyacrylic acid with epoxy functional groups, acrylic polymer with epoxy functional groups polyacrylic acid with anionic/amino C6 spacer functional groups, polyacrylic acid with anionic/tertiary amine functional groups, polystyrene with anionic/quaternary amine functional groups, polystyrene with cationic/sulphonic functional groups, polyacrylic acid with carboxylic ester functional groups, polystyrene with phenyl functional groups, polymethacrylate with octadecyl functional groups, polystyrene with styrene/methyl functional groups, magnetic silica particles with Ni-NTA functional group, or magnetic nanoparticles with a core of magnetite and a dextran shell with Ni-NTA functional group, macroporous resins or beads of macroporous styrene or styrene/methacrylate. While, in principle, any suitable solid support known in the art can be used in the inventive method, Ni agarose beads or Ni NTA agarose resins are not preferred for the reasons as set forth above. Exemplary solid supports useful for immobilizing the enzymes used in the inventive method include, but are not limited to, Sepabeads / ReliZyme (Resindion): EC-EP, including EC-EP/S and EC-EP/M, EP403/M, EP403/S HFA403M, HFA403S, HG403, EP400/SS EC-HG, EC-HFA, EC-EA/M, EA403/S and EC-HA including EC-HA/S and EC-HA/M; Immobeads (ChiralVision) Imm150P, IB-COV1, IB-COV2, IB-COV3, IB-ANI1, IB-ANI2, IB-ANI3, IB-ANI4, IB-CAT1, IB-ADS1, IB-ADS2, IB-ADS3 and IB-ADS4, IB-CAT-1, IB-ANI-1, IB-ANI-2, IB-ANI-3, IB-ANI-4; Eupergit (Röhm GmbH & Co. KG) and magnetic particles (micromod GmbH): Nano-mag, Sicastar-6 and Sicastar-1.5, enzyme immobilization resins Lifetech^{™} (Purolite): Epoxy methacrylate: ECR8215, ECR8215F, ECR8215M, ECR8206, ECR8206F, ECR8206M, ECR8204, ECR8204F, ECR8204M, ECR8209, ECR8209F, ECR8209M, ECR8285, ECR8285F, ECR8285M, Amino C2 or C6 methacrylate: ECR8305, ECR8305F, ECR8305M, ECR8309, ECR8309F, ECR8309M, ECR8315, ECR8315F, ECR8315M, ECR8404 ECR8404F, ECR8404M, ECT8409, ECT8409F, ECT8409M, ECR8415, ECR8415F, ECR8415M, macroporous resins ECR1090, ECR1091, ECR1091M, ECR1061, ECR1030, ECR1030F, ECR8806F; ionic resins ECR1504, ECR1508, ECR1604, ECR1640, and magnetic particles (micromod GmbH): Nano-mag-D and Sicastar-M-CT.

Solid support materials which result in mechanically stable beads or resins with enzymes immobilized thereon are preferred with regard to reuse and/or recycling of the beads or resins for the production of CMP-Neu5Ac and more preferred with regard to a continuous process of the method for production of CMP-Neu5Ac. A mechanically stable solid support is characterized in resistance to abrasion, mechanical stress and is suitable for a high number of cycles, such as at least 10, more preferably at least 12, more preferably at least 14, more preferably at least 16, more preferably at least 18, and most preferably at least 20 cycles. It could be shown that immobilization of enzymes through covalent binding to a solid support provides mechanically stable beads or resins, which has been shown to be particularly suitable for reuse and/or recycling of the resins or beads with immobilized enzymes for the production of CMP-Neu5Ac.

Surprisingly it has been found that with beads or resins comprising a polymer with epoxide functional groups, such as for example, but not limited to polymethacrylate with epoxide functional groups, polymethacrylate with amino epoxide functional groups, polymethacrylate with ethylenediamine functional groups, polymethacrylate with epoxide functional groups and butyl functional groups polyacrylic acid with epoxy functional groups, acrylic polymer with epoxy functional groups, that allow covalent binding of the enzymes to be immobilized, mechanically robust resins or beads may be obtained.

Thus, reusable, mechanically stable solid support in form of beads or resins with enzymes immobilized thereon are preferred with regard to co-immobilization of the set of enzymes from crude cell lysate or crude cell homogenate, and with regard to retaining larges parts of or increasing the activity of all enzymes co-immobilized and with regard to reuse and/or recycling of the beads or resins for the production of CMP-Neu5Ac and with regard to a continuous process of the method for production ofCMP-Neu5Ac. The solid supports are inter alia characterized in resistance to abrasion, mechanical stress and are suitable for a high number of cycles, such as at least 10, more preferably at least 12, more preferably at least 14, more preferably at least 16, more preferably at least 18, and most preferably at least 20 cycles. It could be shown that immobilization of enzymes through covalent binding to a solid support provides mechanically robust beads or resins, which has been shown to be particularly suitable for reuse and/or recycling of the resins or beads with immobilized enzymes for the production of CMP-Neu5Ac, which allows the co-immobilization of the set of enzymes from crude cell lysate and which retains large parts of or increases the activity of all enzymes co-immobilized. Surprisingly it has been found that with beads or resins comprising epoxide functional groups, amino epoxide functional groups, ethylenediamine functional groups, or epoxide functional groups and a hydrophobic group, such as butyl, octyl, methyl, phenyl, butyl functional groups that allow covalent binding of the enzymes to be immobilized, robust solid resins or beads may be obtained.

Epoxy-activated resins or beads allow multipoint covalent binding between an enzyme and the resin or bead. Preferably the resin backbone is composed of methacrylate with porosities of 0.01 nm to 10000 nm or 0.1 Å to 100000 Å. In a preferred embodiment the porosity of an epoxy functionalized resin or bead, for example an epoxy methacrylate resin or bead, may be 30 nm to 60 nm. In a preferred embodiment the porosity of an epoxy methacrylate resin or bead may be 40nm to 60nm. In a preferred embodiment the porosity of an epoxy functionalized resin or bead, for example an epoxy methacrylate resin or bead, may be 50 nm to 60 nm. In a preferred embodiment the porosity of an epoxy functionalized resin or bead, for example an epoxy methacrylate resin or bead, may be 60 nm to 120 nm. In a preferred embodiment the porosity of an epoxy functionalized resin or bead, for example an epoxy methacrylate resin or bead, may be 120 nm to 180 nm. The epoxy functionalized resin or bead, for example an epoxy methacrylate resin or bead, may form very stable covalent linkages with different protein groups, such as amino, thiol, phenolic, preferably under very mild pH and temperature conditions. The resins are preferably mechanically stable and the resin with immobilized enzymes may be preferably used in a stirred tank or column reactor.

Amino resins, such as amino C2 functionalized resins or amino C6 functionalized resins or other amino resins such as amino C3, amino C4, amino C5, amino C7 and so on, such as for example but not limited to amino C2 methacrylate resins or amino C6 methacrylate resins may pre-activated, for example by glutaraldehyde and then used in the covalent immobilization of enzyme. Reaction of the aldehyde groups with amino groups of enzymes form Schiff bases which results in multipoint covalent binding. A linkage may be also achieved by reduction with borohydrides. Thus a reversible immobilization may become irreversible by means of crosslinking step: the enzyme may be adsorbed onto the carrier and then crosslinked by using, for example, glutaraldehyde. The crosslinked enzyme or the crosslinked enzyme may cover the carrier like a net. Amino functionalized resins, such as amino C2 methacrylate resins or amino C6 methacrylate resins have preferably porosities in the range of 30nm to 180nm or 300Å to 1800Å. In a preferred embodiment the porosity of an amino functionalized resin, such as amino C2 methacrylate resin or bead or of an amino C6 methacrylate resin or bead may be 30nm to 60nm. In a preferred embodiment the porosity of an amino functionalized resin, such as an amino C2 methacrylate resin or bead or of an amino C6 methacrylate resin or bead may be 60nm to 120nm. In a preferred embodiment the porosity of an amino functionalized resin, such as an amino C2 methacrylate resin or bead or of an amino C6 methacrylate resin or bead may be 120nm to 180nm.

Another method for irreversible immobilization is the activation of hydroxyl functional groups, such as for example for 1,2-diol-functionalized resins or beads.

Thus, particularly preferred are beads or resins comprising polymethacrylate with epoxide functional groups and polymethacrylate with amino epoxide functional groups. Preferably the beads or resins comprising polymethacrylate with epoxide functional groups are hydrophilic. Covalent enzyme immobilization is particularly preferred. In preferred embodiments the beads or resins are not functionalized with apolar groups such as butyl or octadecyl groups. In preferred embodiments the resins or beads are hydrophilic.

Preferably, the methacrylate polymer has the form of beads. Preferably, the beads have a particle size in the range of 150 µm - 300 µm. Preferably, the methacrylate polymer is porous with a pore diameter between 600 Å - 1200 Å. In one embodiment, the methacrylate polymer is of low porosity having a pore diameter between 300 Å - 600 Å. In one embodiment, the methacrylate polymer is of low porosity having a pore diameter between 450 Å - 650 Å. In one embodiment, the methacrylate polymer is of high porosity having a pore diameter between 1200 Å - 1800 Å. In one embodiment, the methacrylate polymer is further functionalized with butyl groups. In one embodiment, the methacrylate polymer is further functionalized with a hydrophobic group such as butyl, methyl, phenyl, octyl.

Preferably, the solid support is composed of a resin or beads selected from: sepabeads (Resindion): EC-EP, EP403/M, EP403/S, HFA403, EA403, HA403, EC-EA/M and EC-HA, ; immobeads (ChiralVision) IB-COV1, IB-COV2, IB-COV3, IB-ANI1, IB-ANI1, IB-CAT1, Eupergit^{®} (Röhm GmbH & Co. KG), enzyme immobilization resins (Purolite): Epoxy methacrylate: ECR8215, ECR8215F, ECR8215M, ECR8206, ECR8206F, ECR8206M, ECR8204, ECR8204F, ECR8204M, ECR8209, ECR8209F, ECR8209M, ECR8285, ECR8285F, ECR8285M, Amino C2 or C6 methacrylate: ECR8305, ECR8305F, ECR8305M, ECR8309, ECR8309F, ECR8309M, ECR8315, ECR8315F, ECR8315M, ECR8404 ECR8404F, ECR8404M, ECT8409, ECT8409F, ECT8409M, ECR8415, ECR8415F, ECR8415M.

Preferably, the solid support is composed of a resin or beads selected from: sepabeads (Resindion): EC-EP, EP403, EP403/M, EP403/S, EC-HFA, HFA403, HFA403/M, HFA 403/S, immobeads (ChiralVision) IB-COV2, IB-COV3, (Purolite) ECR8215, ECR8215F, ECR8215M, ECR8204F, ECR8204M, ECR8204, ECR8209F, ECR8209M, ECR8209, Eupergit^{®} (Röhm GmbH & Co. KG).

Optionally, the set of enzymes further comprises an inorganic diphosphatase (PPA). Additionally, the set of enzymes further comprises a one-domain polyphosphate kinase 2 (1D-PPK2) and/or a two-domain polyphosphate kinase 2 (2D-PPK2). The inorganic diphosphatase (PPA), the one-domain polyphosphate kinase 2 (1DPPK2) and/or the two-domain polyphosphate kinase 2 (2DPPK2) are preferably also co-immobilized with the above-mentioned enzymes on the same solid support.

Preferably, the set of enzymes is immobilized or co-immobilized on a polymer through covalent bonds.

Preferably, the set of enzymes comprises an N-acylglucosamine 2-epimerase (AGE), an N-acetyl-neuraminate lyase (NAL), an N-acylneuraminate cytidylyltransferase (CSS), optional a uridine kinase (UDK), a uridine monophosphate (UMP) kinase, a polyphosphate kinase 3 (PPK3), and a β-galactoside α-2,6-sialyltransferase consisting of an amino acid sequence as set forth in SEQ ID NO: 1 or an amino acid sequence with at least 80% identity to SEQ ID NO: 1, wherein the set of enzymes is preferably co-immobilized on a polymer functionalized with epoxy groups.

Preferably, the set of enzymes of the present invention further comprises an inorganic diphosphatase (PPA), a one-domain polyphosphate kinase 2 (1D-PPK2) and/or a two-domain polyphosphate kinase 2 (2D-PPK2).

Preferably, in the method of the present invention, the resulting solution in the step B) has a pH value in a range of 5.0 - 10.0, more preferred 5.5 - 9.5, and most preferred 6.0 - 9.0, still more preferred 6.5 - 9.0, most preferred 7.0 - 9.0.

Preferably, in the method of the present invention, the concentration of *N*-acetyl-D-glucosamine (GlcNAc) is in the range of 10 mM to 3500 mM, preferred 20 mM to 3000 mM, more preferred 15 mM to 2500 mM, still more preferred 20 mM to 2000 mM, most preferred is in the range of 20 mM to 1000 mM;
and/or the concentration of the pyruvate is in the range of 10 mM to 3500 mM, preferred 20 mM to 3000 mM, more preferred 15 mM to 2500 mM, still more preferred 20 mM to 2000 mM, most preferred is in the range of 20 mM to 1000 mM;
and/or the concentration of the cytidine is in the range of 1 mM to 50 mM, preferred 1 mM to 40 mM, more preferred 1 mM to 30 mM, still more preferred 10 mM to 20 mM, most preferred is in the range of 1 mM to 15 mM;
and/or the concentration of adenosine 5'-triphosphate (ATP) is in the range of 0.001 mM to 10 mM, preferred 0.005 mM to 10 mM, more preferred 0.01 mM to 10 mM, still more preferred 0.05 mM to 10 mM, most preferred is in the range of 0.1 mM to 10 mM;
and/or the concentration of polyphosphate is in the range of 1 mM to 30 mM, preferred 1 mM to 25 mM, more preferred 1 mM to 20 mM, still more preferred 1 mM to 15 mM, most preferred is in the range of 1 mM to 10 mM.

Preferably, in the method of the present invention, the ratio of the *N*-acetyl-D-glucosamine and the cytidine is in the range of 1:1 to100 to 1.

Preferably, in the method of the present invention, the resulting solution further comprises Mg²⁺ with a concentration in the range of 0.1 mM to 500 mM, preferred 0.1 mM to 200 mM, more preferred 1 mM to 100 mM, still more preferred 10 mM to 100 mM, most preferred 20 mM to 50 mM.

Preferably, in the method of the present invention, each of the enzymes has the following amino acid sequence:
the N-acylglucosamine 2-epimerase (AGE) comprises an amino acid sequence as set forth in SEQ ID NO: 10;
the N-acetylneuraminate lyase (NAL) comprises an amino acid sequence as set forth in SEQ ID NO: 11;
the N-acylneuraminate cytidylyltransferase (CSS) comprises an amino acid sequence as set forth in SEQ ID NO: 12;
optional the uridine kinase (UDK) comprises an amino acid sequence as set forth in SEQ ID NO: 13;
the uridine monophosphate kinase (URA6) comprises an amino acid sequence as set forth in SEQ ID NO: 14;
the polyphosphate kinase 3 (PPK3) comprises an amino acid sequence as set forth in SEQ ID NO: 15; and
the inorganic diphosphatase (PPA) comprises an amino acid sequence as set forth in SEQ ID NO: 16.

In particular, the solid support is composed of beads or resins comprising a polymer with epoxide functional groups, with amino epoxide functional groups, with ethylenediamine functional groups, with amino C2 functional groups, with amino C6 functional groups, with anionic/amino C6 spacer functional groups. The solid support is a porous or a non-porous particle including nanoparticle or a porous bead having a pore size of 0.1 Å to 100000 Å.

Preferably, the set of enzymes is directly co-immobilized on a solid support from cell lysate or cell homogenate.

An appropriate concentration of Mg²⁺ as cofactor contributes full activation of the uridine monophosphate (UMP) kinase.

In some embodiments, the resulting solution in the method of the present invention further comprises Mg²⁺ with a concentration in a range of 0.1 mM to 500 mM, 0.1 mM to 200 mM, preferably 10 to 100 mM, more preferably, 50 to 100 mM, most preferably 20 mM to 50 mM. Preferably, a source of Mg²⁺ is magnesium bromide, magnesium chloride, magnesium carbonate, monomagnesium phosphate, magnesium phosphate, magnesium sulfate and hydrates thereof.

Optionally, the resulting solution further comprises a reducing reagent such as 2-mercaptoethanol and dithiothreitol (DTT).

The reaction temperature of the reaction solution affects the efficiency of the enzymatic cascade reactions. Therefore, in the methods of the present invention, the optimal reaction temperature of the step B) is in a range of 5°C to 65°C, preferred, 10°C to 60°C, still preferred 15°C to 57°C, more preferred 20°C to 55°C, still more preferred 25°C to 53°C, still more preferred 30°C to 51°C, and most preferred 34°C to 50°C.

In the present invention, a concentration of *N*-acetyl-D-glucosamine is in a range of 1 mM to 5000 mM, preferred 1 mM to 4000 mM, more preferred 2 mM to 4500 mM, still more preferred 5 mM to 3000 mM, most preferred 10 mM to 2000 mM; and/or a concentration of the pyruvate is in a range of 1 mM to 5000 mM, preferred 2 mM to 4000 mM, more preferred 5 mM to 3000 mM, still more preferred 10 mM to 3000 mM, most preferred 20 mM to 2000 mM; and/or a concentration of the cytidine is in a range of 0.1 mM to 2000 mM, preferred 1 mM to 1000 mM, more preferred 1 mM to 500 mM, and/or a concentration of adenosine 5'-triphosphate (ATP) is in a range of 0.001 mM to 100 mM, preferred 0.01 mM to 100 mM, more preferred 0.1 mM to 500 mM, still more preferred 0.1 mM to 100 mM, most preferred 0.1 mM to 40 mM:

### In vivo preparation of α-sialyl-β-D-galactoside saccharides

Another aspect of the present invention is the *in vivo* production of α-sialyl-β-D-galactoside saccharides using genetically engineered cells expressing the inventive β-galactoside α-sialyltransferase.

Therefore, the present invention is also directed to an *in vivo* method for producing an α-sialyl-β-D-galactoside saccharide, comprising
A') providing a solution comprising a β-D-galactoside saccharide, optionally a sialic acid donor, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-D-galactoside saccharide,
   wherein the solution is a fermentation broth further comprising at least one carbon source.

Preferably, the at least one carbon source is sucrose.

Suitable fermentation conditions for sialyltransferase expression are, for example: E.coli cell growth at 37°C in a volume of 200 mL at a shaking speed of 180 - 250 rpm in 1 L shake flasks. The media can for example be LB or TB media with antibiotics according to the resistance marker on the plasmid. Reducing the temperature to 16-30°C at an OD600 of 0.4 to 0.8 and inducing with 0.1 - 1 mM of IPTG. Harvesting the enzyme, between 4-16 hours post-induction.

In a preferred embodiment, the cell comprises a nucleic acid encoding an enzyme with β-galactoside α-sialyltransferase activity consists of an amino acid sequence with at least 80% identity to SEQ ID NOs: 4-9, more preferably with at least 85% identity to SEQ ID NOs: 4-9, more preferably with at least 90% identity to SEQ ID NOs: 4-9, more preferably at least 92% identity to SEQ ID NOs: 4-9, more preferably with at least 93% identity to SEQ ID NOs: 4-9, more preferably with at least 94% identity to SEQ ID NOs: 4-9, more preferably with at least 95% identity to SEQ ID NOs: 4-9, more preferably with at least 96% identity to SEQ ID NOs: 4 - 9, more preferably with at least 97% identity to SEQ ID NOs: 4 - 9, more preferably with at least 98% identity to SEQ ID NOs: 4-9, and most preferably with at least 99% identity to SEQ ID NOs: 4-9.

In another preferred embodiment, the *in vivo* method for producing an α-sialyl-β-D-galactoside saccharide, comprising
A') providing a solution comprising a β-D-galactoside saccharide, optionally a sialic acid donor, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside saccharide,
   wherein the solution is a fermentation broth further comprising at least one carbon source.

In a preferred embodiment, the β-D-galactoside saccharide is a glycopeptide, a glycoprotein, a glycolipid, a glycan or a human milk oligosaccharide (HMO). Reworded, the present invention is directed to an *in vivo* method for producing an α-sialyl-β-D-galactoside glycopeptide, glycoprotein, glycolipid, glycan or human milk oligosaccharide, comprising
A') providing a solution comprising a β-D-galactoside glycopeptide, glycoprotein, glycolipid, glycan or human milk oligosaccharide, optionally a sialic acid donor, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside glycopeptide, glycoprotein, glycolipid, glycan or human milk oligosaccharide,
   wherein the solution is a fermentation broth further comprising at least one carbon source.

Preferably, the *in vivo* method for producing an α-sialyl-β-D-galactoside glycopeptide, glycoprotein, glycolipid, glycan or human milk oligosaccharide, comprises
A') providing a solution comprising a β-D-galactoside glycopeptide, glycoprotein, glycolipid, glycan or human milk oligosaccharide, optionally a sialic acid donor, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside glycopeptide, glycoprotein, glycolipid, glycan or human milk oligosaccharide,
   wherein the solution is a fermentation broth further comprising at least one carbon source.

In a preferred embodiment, the β-D-galactoside saccharide comprises one or more terminal β-D-galactose residues.

Reworded, the present invention is directed to an *in vivo* method for producing an α-sialyl-β-D-galactoside saccharide, comprising
A') providing a solution comprising a β-D-galactoside saccharide, optionally a sialic acid donor, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside saccharide,
   wherein the solution is a fermentation broth further comprising at least one carbon source and wherein the β-D-galactoside saccharide comprises one or more terminal β-D-galactose residues.

In a preferred embodiment, the α-sialyl-β-D-galactoside saccharide is a sialylated human milk oligosaccharide selected from 6'-siallylactose, 3'-siallylactose, sialyllacto-N-tetraose a (LSTa), sialyllacto-N-neotetraose d (LSTd), sialyllacto-N-neotetraose c (LSTc), sialyllacto-N-tetraose b (LSTb) and disialyllacto-N-tetraose (DSLNT).

Reworded, the present invention is directed to an *in vivo* method for producing an α-sialyl-β-D-galactoside saccharide, comprising
A') providing a solution comprising a β-D-galactoside saccharide, optionally a sialic acid donor, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-(2→6)-β-D-galactoside saccharide,
   wherein the solution is a fermentation broth further comprising at least one carbon source, and wherein the α-sialyl-(2→6)-β-D-galactoside human milk oligosaccharide is selected from 6'-siallylactose, 3'-siallylactose, sialyllacto-N-tetraose a (LSTa), sialyllacto-N-neotetraose d (LSTd), sialyllacto-N-neotetraose c (LSTc), sialyllacto-N-tetraose b (LSTb) and disialyllacto-N-tetraose (DSLNT).

Preferably, the α-sialyl-β-D-galactoside human milk oligosaccharide is 6'-siallylactose.

Another embodiment is directed to an *in vivo* method for producing an α-sialyl-β-D-galactoside glycoprotein, comprising
A') providing a solution comprising a β-D-galactoside glycoprotein comprising A2G2 glycan, optionally a sialic acid donor, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside glycoprotein comprising A2G2S1 and/or A2G2S2,
   wherein the solution is a fermentation broth further comprising at least one carbon source.

Another embodiment is directed to an *in vivo* method for producing an α-sialyl-β-D-galactoside monoclonal antibody, comprising
A') providing a solution comprising a β-D-galactoside monoclonal antibody, optionally a sialic acid donor, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside monoclonal antibody,
   wherein the solution is a fermentation broth further comprising at least one carbon source.

In a preferred embodiment, the sialic acid donor is a sialic acid nucleotide, preferably CMP-Neu5Ac. Thus, the present invention is directed to an *in vivo* method for producing an α-sialyl-β-D-galactoside saccharide, comprising
A') providing a solution comprising a β-D-galactoside saccharide, CMP-Neu5Ac, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside saccharide,
   wherein the solution is a fermentation broth further comprising at least one carbon source.

In another preferred embodiment, the *in vivo* method for producing an α-sialyl-β-D-galactoside saccharide, comprises
A') providing a solution comprising a β-D-galactoside saccharide, CMP-Neu5Ac, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside saccharide,
   wherein the solution is a fermentation broth further comprising at least one carbon source.

In a preferred embodiment, the *in vivo* method for producing an α-sialyl-β-D-galactoside glycopeptide, glycoprotein, glycolipid, glycan or human milk oligosaccharide, comprising
A') providing a solution comprising a β-D-galactoside glycopeptide, glycoprotein, glycolipid, glycan or human milk oligosaccharide, optionally a sialic acid donor, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside glycopeptide, glycoprotein, glycolipid, glycan or human milk oligosaccharide,
   wherein the solution is a fermentation broth further comprising at least one carbon source.

Preferably, the *in vivo* method for producing an α-sialyl-β-D-galactoside glycopeptide, glycoprotein, glycolipid, glycan or human milk oligosaccharide, comprises
A') providing a solution comprising a β-D-galactoside glycopeptide, glycoprotein,
   glycolipid, glycan or human milk oligosaccharide, CMP-Neu5Ac, and a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside glycopeptide, glycoprotein, glycolipid, glycan or human milk oligosaccharide,
   wherein the solution is a fermentation broth further comprising at least one carbon source.

In a preferred embodiment, the *in vivo* method for producing an α-sialyl-β-D-galactoside saccharide, comprises
A') providing a solution comprising a β-D-galactoside saccharide, CMP-Neu5Ac, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside saccharide,
   wherein the solution is a fermentation broth further comprising at least one carbon source and wherein the β-D-galactoside saccharide comprises one or more terminal β-D-galactose residues.

In a preferred embodiment, the *in vivo* method for producing an α-sialyl-β-D-galactoside human milk oligosaccharide, comprises
A') providing a solution comprising a β-D-galactoside human milk oligosaccharide, CMP-Neu5Ac, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside saccharide,
   wherein the solution is a fermentation broth further comprising at least one carbon source and wherein the α-sialyl-β-D-galactoside human milk oligosaccharide is selected from 6'-siallylactose, 3'-siallylactose, sialyllacto-N-tetraose a (LSTa), sialyllacto-N-neotetraose d (LSTd), sialyllacto-N-neotetraose c (LSTc), sialyllacto-N-tetraose b (LSTb) and disialyllacto-N-tetraose (DSLNT).

Preferably, the α-sialyl-β-D-galactoside human milk oligosaccharide is 6'-siallylactose.

Another embodiment is directed to an *in vivo* method for producing an α-sialyl-β-D-galactoside glycoprotein, comprising
A') providing a solution comprising a β-D-galactoside glycoprotein comprising A2G2 glycan, CMP-Neu5Ac, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside glycoprotein comprising A2G2S1 and/or A2G2S2,
   wherein the solution is a fermentation broth further comprising at least one carbon source .

Another embodiment is directed to an *in vivo* method for producing an α-sialyl-β-D-galactoside monoclonal antibody, comprising
A') providing a solution comprising a β-D-galactoside monoclonal antibody comprising A2G2 glycan, CMP-Neu5Ac, and
   a genetically engineered cell comprising a recombinant nucleic acid encoding an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B') mixing said solution and said enzyme and cultivating the genetically engineered cell to produce the α-sialyl-β-D-galactoside monoclonal antibody,
   wherein the solution is a fermentation broth further comprising at least one carbon source .

In some embodiments, the resulting fermentation broth in the step B) has a pH value in a range of 5.0 - 10.0, more preferred 5.5 - 9.5, and most preferred 6.0 - 9.0.

Preferably, the resulting fermentation broth is a buffer solution having a pH value in a range of 5.0 - 10.0, more preferred 5.5 - 9.5, and most preferred 6.0 - 9.0. Preferably, the buffer cell-free reaction is an ammonium bicarbonate solution, a potasium phosphate (Gomori) buffer, or the like having a pH value in a range of 6.0 - 9.0.

### Description of the Figures:

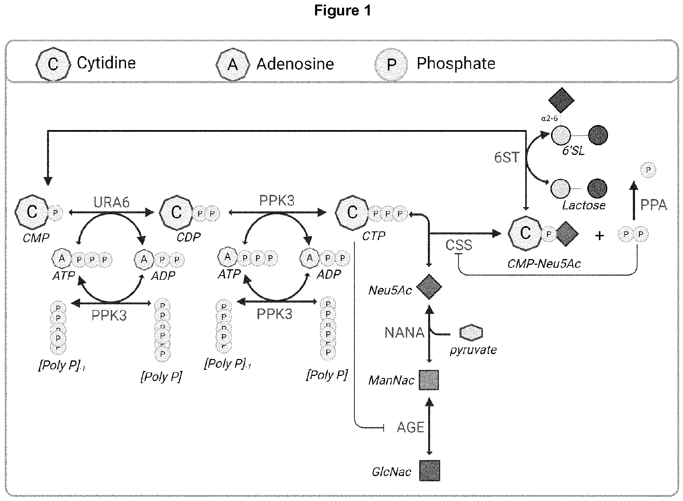

**Figure 1** shows the multi-enzyme cascade through which 6'-SL is enzymatically synthesized in a one pot from lactose and CMP-Neu5Ac formed *in situ* from low-cost substrates *N*-acetyl-D-glucosamine (GlcNAc), pyruvate, polyphosphate and CMP. The reaction cascade comprises (a) the formation of *N*-acetyl-D-mannosamine (ManNAc) from *N*-acetyl-D-glucosamine (GlcNAc), (b) the formation of *N*-acetyl-D-neuraminic acid (Neu5Ac) from ManNAc and pyruvate, (c) the formation of cytidine 5'-diphosphate (CDP) from CMP and ATP, (d) the formation of cytidine 5'-triphosphate (CTP) from CDP and polyphosphate (PolyPₙ), (e) the reaction of Neu5Ac with CTP to CMP-Neu5Ac, and (f) the formation of 6'-sialyllactose from lactose and CMP-Neu5Ac. Optionally, the cascade can be extended by adding a 1D-PPK2 to assist the conversion of ADP to ATP. Also, the cascade can be extended by adding a 2D-PPK2 in order to activate phosphorylation of AMP to ADP. Moreover, the cascade can be extended by adding a 1D-PPK2 and a 2D-PPK2 in order to inhibit frequent hydrolysis of adenosine phosphates.

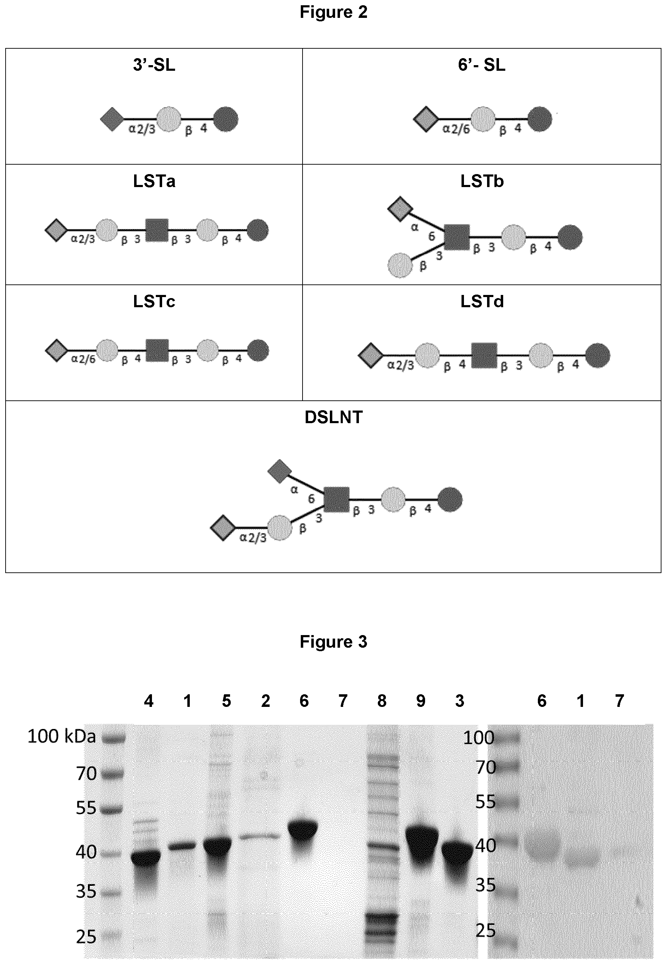

**Figure 2** shows structures of exemplarily sialylated saccharides produced with the inventive method.
**Figure 3** shows a SDS page of the purified sialyltransferases of SEQ ID Nos: 1 - 9. Numbers 1 - 9 correspond to the SEQ ID No.

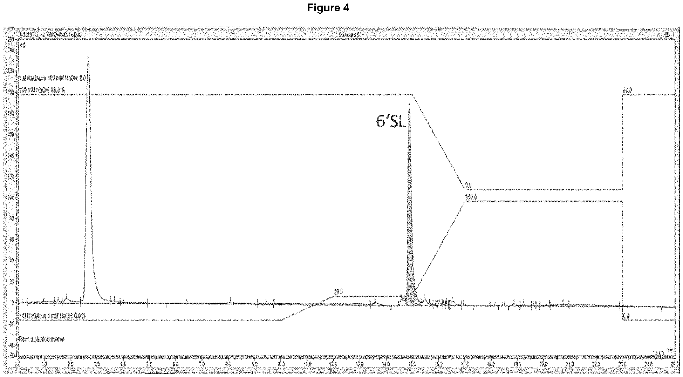

**Figure 4** shows a HPAEC-PAD chromatogram of the reaction mixture of lactose together with *N*-acetyl-D-glucosamine (GlcNAc), pyruvate, sialyltransferase 1, polyphosphate and CMP, which react *in situ* to CMP-Neu5Ac, after 24 hours. A peak of 6'-SL is detectable at 15 minutes with the applied gradient.

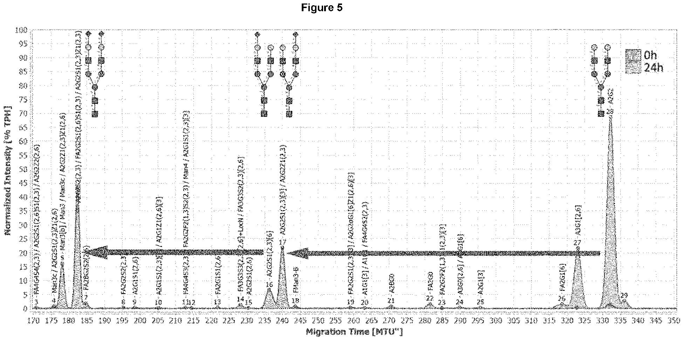

**Figure 5** shows an electropherogram of the reaction product of the sialylation of A2G2 with CMP-Neu5Ac in the presence of the inventive sialyltransferase 7at 0 hour reaction time (dark grey) and 24h hours reaction time (light grey).

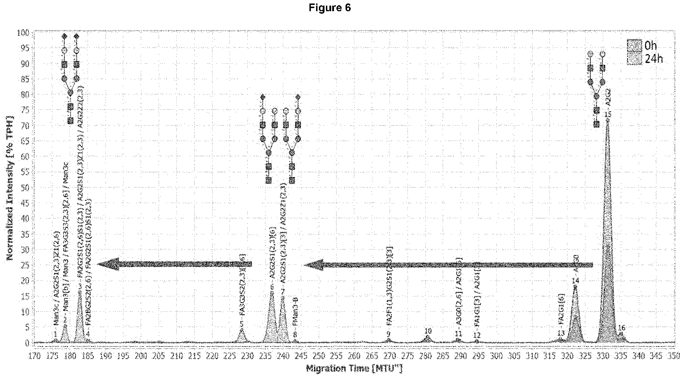

**Figure 6** shows an electropherogram of the reaction product of the sialylation of A2G2 with CMP-Neu5Ac in the presence of the prior art sialyltransferase 3 at 0 hour reaction time (dark grey) and 24h hours reaction time (light grey).

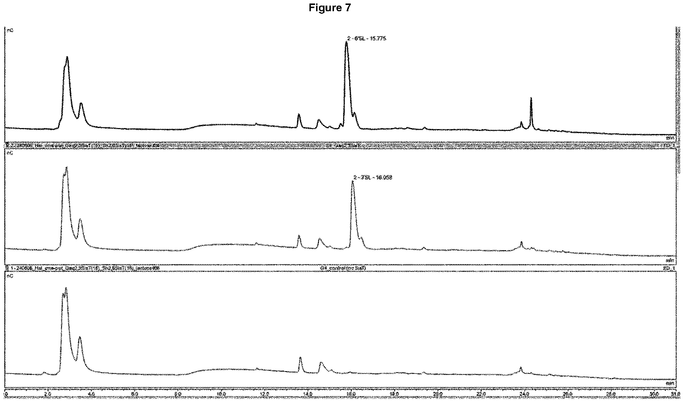

**Figure 7** shows HPAEC-PAD chromatograms of the reaction mixtures of lactose together with *N*-acetyl-D-glucosamine (GlcNAc), pyruvate, polyphosphate, sialyltransferase and CMP, which react *in situ* to CMP-Neu5Ac, after 7.5 hours. sialyltransferases from top to bottom: 5 and 9. 6'-SL (15.8 min) or 3'-SL (16.1 min) was detected at the main product. Bottom is negative control without sialyltransferase.

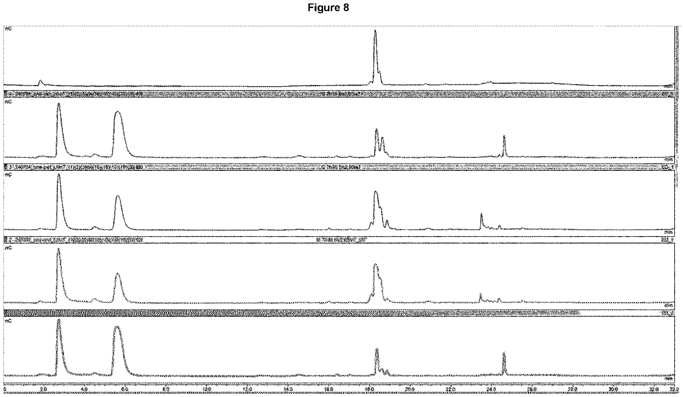

**Figure 8** shows HPAEC-PAD chromatograms of the reaction mixtures of LNnT together with *N*-acetyl-D-glucosamine (GlcNAc), pyruvate, polyphosphate, α-2,6-sialyltransferases and CMP, which react *in situ* to CMP-Neu5Ac, after 7.5 hours. α-2,6-sialyltransferases from top to bottom: of LSTc standard, 4, 1, 5, and 2. The peak of LSTc (18.3 min) was detected as the main product.

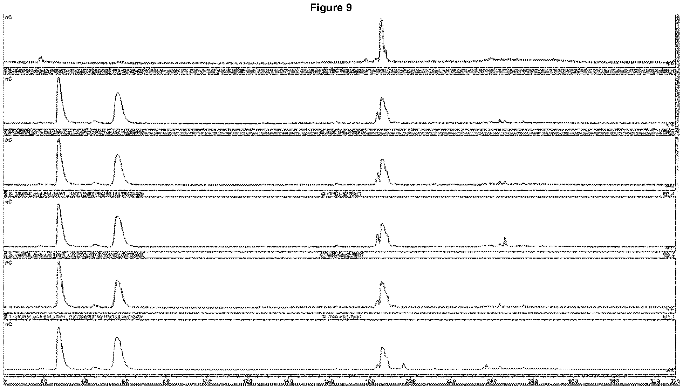

**Figure 9** shows HPAEC-PAD chromatograms of the reaction mixtures of LNnT together with *N*-acetyl-D-glucosamine (GlcNAc), pyruvate, polyphosphate, α-2,3-sialyltransferases and CMP, which react *in situ* to CMP-Neu5Ac, after 7.5 hours. α-2,3-sialyltransferases from top to bottom: LSTd standard, 6, 7, 8, 9 and 3. The peak of LSTd (18.6 min) was detected as the main product.

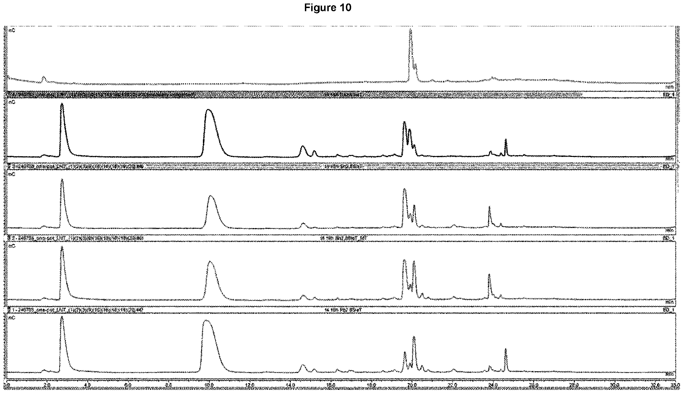

**Figure 10** shows HPAEC-PAD chromatograms of the reaction mixtures of LNT together with *N*-acetyl-D-glucosamine (GlcNAc), pyruvate, polyphosphate, α-2,6-sialyltransferase and CMP, which react *in situ* to CMP-Neu5Ac, after 16 hours. α-2,6-sialyltransferases from top to bottom: LSTa standard, 4, 1, 5, and 2. The peak of LSTb product (20.0 min) is expected to be close to LSTa analogue (19.9 min). Other significant peaks of sialylated-LNT were also observed (19.6 min, sialyltransferases 4, 1, 5; 23.8 min, sialyltransferases 1, 5).

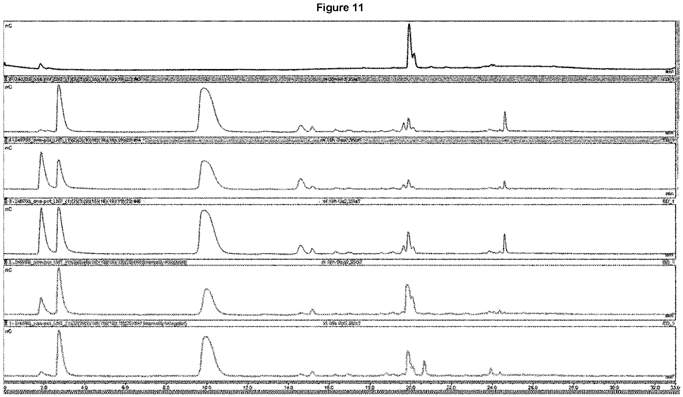

**Figure 11** shows HPAEC-PAD chromatograms of the reaction mixtures of LNT together with *N*-acetyl-D-glucosamine (GlcNAc), pyruvate, polyphosphate, α-2,3-sialyltransferases, and CMP, which react *in situ* to CMP-Neu5Ac, after 16 hours. α-2,3-sialyltransferases from top to bottom: LSTa standard, 6, 7, 8, 9 and 3. The peak of LSTa (19.9 min) was detected as the main product.

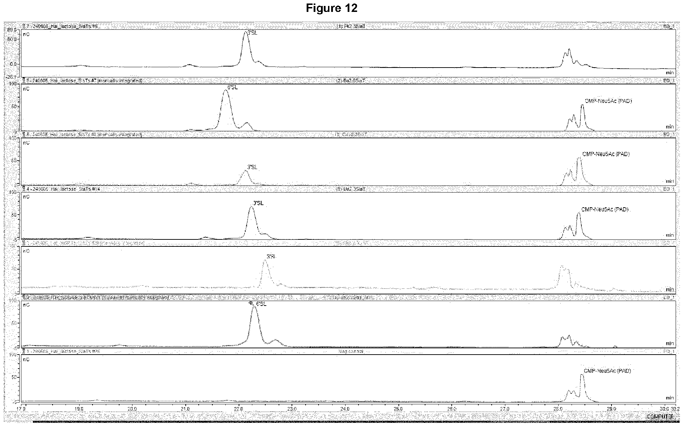

**Figure 12** shows HPAEC-PAD chromatograms of the reaction mixtures of the sialylation of lactose with CMP-Neu5Ac in the presence of the inventive sialyltransferases after 24h. Sialyltransferases from top to bottom: 6; 4; 7; 8; 9; 5; and negative control (without enzyme). The formation of 3'SL or 6'SL product confirmed the sialyltransferase activity; the remaining CMP-Neu5Ac was observed with sialyltransferases 4, 7, and 8.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### Abbreviations and Acronyms

- AGE: *N*-acetyl-D-glucosamine epimerase/isomerase
- ADP: adenosine 5'-diphosphate
- AMP: adenosine 5'-monophosphate
- ATP: adenosine 5'-triphosphate
- dH₂O: deionized water
- CMP: cytidine 5'-monophosphate
- CDP: cytidine 5'-diphosphate
- CTP: cytidine 5'-triphosphate
- CSS: N-acylneuraminate cytidylyltransferase
- *Nm*CSS: N-acylneuraminate cytidylyltransferase of *Neisseria meningitidis serogroup B (strain MC58)*
- NAL: N-acetylneuraminate lyase, or N-acetylneuraminate pyruvate lyase
- GlcN: D-glucosamine
- GlcNAc: *N*-acetyl-D-glucosamine
- ManNAc: *N*-acetyl-D-mannosamine
- Neu5Ac: *N*-acetyl-D-neuraminic acid, or 5-(acetylamino)-3,5-dideoxy-D-*glycero*-α-D-*galacto*-non-2-ulopyranosonic acid
- CMP-Neu5Ac: cytidine 5'-monophosphate *N*-acetyl-D-neuraminic acid
- PolyP: polyphosphate
- PPi: pyrophosphate
- Pi: phosphate
- PPK2: polyphosphate kinase 2
- PPK3: polyphosphate kinase 3
- 1D-PPK2: 1-domain polyphosphate kinase 2, polyphosphate:ADP phosphotransferase
- 2D-PPK2: 2-domain polyphosphate kinase 2, polyphosphate:AMP phosphotransferase
- URA6: uridine monophosphate kinase
- PPA: inorganic pyrophosphatase
- PmPpA: *Pasteurella multocida* inorganic pyrophosphatase

### Chemicals & Reagents

Unless otherwise stated, all chemicals and reagents were acquired from Sigma-Aldrich and CarboSynth, and were of the highest purity available. Solid supports were obtained from Resindion, ChiralVision, Röhm GmbH & Co. KG and micromod GmbH.

### Example 1 Cloning, expression and purification of sialyltransferase enzymes

The full sequence was analyzed and determined to be truncated and/or mutated. The synthetic gene was codon-optimized for bacterial expression with an N- or C-terminal Hiss-tag sequence and inserted into a corresponding plasmid (Table 1), which was heat-shocked transformed into *E.coli* BL21 DE3. The transformant strain was grown in 200 mL Terrific Broth (TB) liquid medium with shaking at 150 rpm or on LB agar plates at 37 °C. Media were supplemented with 100 µg·mL-1 ampicillin or 50 pg·mL-1 kanamycin. The expression was induced by addition of isopropyl β-d-1-thiogalactopyranoside (IPTG, final concentration 0.5 mM) at an OD₆₀₀ of 0.8-1.2, and the cultivation was continued for 16 h at 18 °C. Cells were harvested by centrifugation (4000 × g, 10 min, 4 °C) and stored at -20 °C until further use. The cell pellet was thawed on ice and resuspended in buffer A (20 mM sodium phosphate buffer, 0.5 M sodium chloride, pH 7.6). Cells were lysed using high-pressure homogenization (Maximator GmbH, Germany) at 1,350 bar. After centrifugation for 30 min at 20 000 × g, the supernatant was passed through a 0.2 µm sterile filter, before loading on a 5 mL His-trap HP columns (GE Life Sciences, USA) installed in an ÄKTA purifier system (Amersham Bioscience, USA) at a flow rate of 5 mL·min-1. After washing out unbound protein using 5 column volumes (CV) of buffer A mixed with 4% v/v buffer B (20 mM sodium phosphate buffer, 0.5 M NaCl, 0.5 M imidazole, pH 7.6), the retained protein was eluted by applying a 25 CV linear gradient towards 60% buffer B at a flow rate of 5 mL·min-1. The fractions containing the enzyme were pooled and dialyzed in 5 L of buffer C (20 mM sodium phosphate buffer, pH 7.6). The protein concentrations were determined with bicinchoninic acid (BCA) assay using the Pierce^{™} BCA Protein Assay Kit (ThermoFisher Scientific). The purified enzymes were analyzed with SDS-PAGE with 10% precast gels (BIORAD Laboratories, Inc.) and Coomassie staining.

**Table 1: Sialyltransferases, plasmids and enzyme production yields**

| **Enzyme** | **SEQ ID** | **Vector** | **Hiss-tag position** | **Selection marker** | **Enzyme production [mg/ L culture]** |
|---|---|---|---|---|---|
| Sh2,6SiaT | 1 | pET-22b(+) | C-terminal | Ampicilin | 16 |
| Pb2,6SiaT | 2 | pET-28a(+) | N-terminal | Kanamycin | 2 |
| Pp2,3SiaT | 3 | pET-28a(+) | N-terminal | Kanamycin | 650 |
| Ba2,6SiaT | 4 | pET-22b(+) | C-terminal | Ampicilin | 23 |
| Sh2,6SiaT_MT | 5 | pET-22b(+) | C-terminal | Ampicilin | 96 |
| Pk2,3SiaT | 6 | pET-22b(+) | C-terminal | Ampicilin | 307 |
| Gau2,3SiaT | 7 | pET-22b(+) | C-terminal | Ampicilin | 175 |
| Ua2,3SiaT | 8 | pET-22b(+) | C-terminal | Ampicilin | 35 |
| Gaq2,3SiaT | 9 | pET-28a(+) | N-terminal | Kanamycin | 254 |

SEQ ID NO. 19: sequence of the construction vector pET-28a(+)
SEQ ID NO. 20: sequence of the construction vector pET-22b(+)

### Example 2: Activity of sialyltransferases

Lactose, LNT, LNnT and A2G2 (GlcNAc₂Man₃GlcNAc₂Gal₂) were used as substrates for the sialyltransferases (SiaT) in the presence of CMP-Neu5Ac as sialic acid donor.

*Reaction conditions* 1: 50 µL reaction mixture contained 1 mM sugar (lactose, LNT or LNnT), 1.5 mM CMP-Neu5Ac, 0.2 mg/mL SiaT, 50 mM NH₄HCO₃ pH 7.8 at 30 °C for 24 hours. The formation of 3'SL or 6'SL by using lactose as the substate confirmed the sialyltransferase activity; the remaining CMP-Neu5Ac was observed with Ba2,6SiaT, Gau2,3SiaT and Ua2,3SiaT, suggesting that these sialyltransferases have no hydrolyse activity towards CMP-Neu5Ac (see Figure 12).

*Reaction conditions 2:* 20 µL reaction mixture contained 0.0125 mg/mL A2G2, 4.0 mM CMP-Neu5Ac, 0.05 mg/mL SiaT, 50 mM MOPS pH 7.0 at 30 °C for 24 hours. Formation of A2G2S1 and A2G2S2 was observed in electropherograms, thereby proving an activity similar to α-2,6-sialytransferases from human as a positive control. Full conversion to A2G2S2 as the main product was observed for sialyltransferases 4, 7, and 8, which do not exhibit any hydrolysis activity towards CMP-Neu5Ac or sialidase activity towards A2G2S1 and A2G2S2 products (see Figure 4), compared to partially converted A2G2S2 as for other SiaTs (see Figure 5).

### Example 3: Hydrolase activity of sialyltransferases:

Sialyltransferase enzymes listed in Table 1 were incubated with 1.5 mM CMP-Neu5Ac, 0.2 mg/mL SiaT, 50 mM NH₄HCO₃ pH 7.8 at 30 °C for 24 hours.

Hydrolysis of CMP-Neu5Ac was observed in the presence of enzymes 1 - 3, 5, 6, 8, and 9 (see Table 2 below).

**Table 2: Screening for hydrolase activity**

| **Enzyme** | **SEQ ID** | **CMP-Neu5Ac hydrolysis** |
|---|---|---|
| Sh2,6SiaT | 1 | yes |
| Pb2,6SiaT | 2 | yes |
| Pp2,3SiaT | 3 | yes |
| Ba2,6SiaT | 4 | no |
| Sh2,6SiaT_MT | 5 | yes |
| Pk2,3SiaT | 6 | yes |
| Gau2,3SiaT | 7 | no |
| Ua2,3SiaT | 8 | no |
| Gaq2,3SiaT | 9 | yes |

### Example 4: Production of Neu5Acylated biomolecules

Adding sialyltransferases to a multi-enzyme cascade for *in-situ* regeneration of CMP-Neu5Ac. In this way, CMP is not needed as an external substrate and is, moreover, continuously recycled. The additional advantage of the latter is that the inherited hydrolysis activity of many sialyltransferases using CMP-Neu5Ac as the starting substrate is bypassed.

**Table 3: Reaction conditions.**

| **Component** | **concentration** | |
|---|---|---|
| NH₄HCO₃ buffer | 150 | mM |
| CMP | 2.5 | mM |
| GlcNAc | 125 | mM |
| ATP | 2.5 | mM |
| PolyP₁₄ | 25 | mM |
| pyruvate | 125 | mM |
| β-D-galactoside saccharide (lactose, LNT or LNnT) | 100 | mM |
| MgCl₂ | 100 | mM |
| Freeze-dried, purified Duet-D cascade | 0.5 | mg/mL |
| Freeze-dried, purified sialyltransferase | 0.2 | mg/mL |

| **Reactions Conditions** | | |
|---|---|---|
| Reaction volume | 50 uL | |
| pH | 7.8 | |
| Temp | 30 °C | |
| Shaking rate | 900 rpm | |

The reaction was analyzed at the start of the reaction and after 6h - 24h with HPAEC-PAD showing that corresponding sialylated-saccharides (see Example 2) were successfully produced (see Figures 7 - 11).

**Table 4: Enzymes used in this example**

| **Enzyme** | **Abbreviation** | **EC class** | **Origin** | **SEQ ID** |
|---|---|---|---|---|
| β-galactoside-α2,6-sialyltransferase | Sh2,6SiaT | 2.4.3.1 previously 2.4.99.1 | *Shewanella halifaxensis* | 1 |
| β-galactoside-α2,6-sialyltransferase | Pb2,6SiaT | 2.4.3.1 previously 2.4.99.1 | *Photobacterium sp. JT-ISH-224* | 2 |
| β-galactoside-α2,3-sialyltransferase | Pp2,3SiaT | 2.4.3.4 previously 2.4.99.4 | *Photobacterium phosphoreum JT-ISH-467* | 3 |
| β-galactoside-α2,6-sialyltransferase | Ba2,6SiaT | 2.4.3.1 previously 2.4.99.1 | *Brevinema andersonii* | 4 |
| β-galactoside-α2,6-sialyltransferase | Sh2,6SiaT_M T | 2.4.3.1 previously 2.4.99.1 | *Shewanella halifaxensis* | 5 |
| β-galactoside-α2,3-sialyltransferase | Pk2,3SiaT | 2.4.3.4 previously 2.4.99.4 | *Photobacterium kishitanii* | 6 |
| β-galactoside-α2,3-sialyltransferase | Gau2,3SiaT | 2.4.3.4 previously 2.4.99.4 | *Glaesserella australis* | 7 |
| β-galactoside-α2,3-sialyltransferase | Ua2,3SiaT | 2.4.3.4 previously 2.4.99.4 | *Ursidibacter arcticus* | 8 |
| β-galactoside-α2,3-sialyltransferase | Gaq2,3SiaT | 2.4.3.4 previously 2.4.99.4 | *Gallalistipes aquisgranensis (Rikenellaceae bacterium)* | 9 |
| AGE family epimerase/isomerase | AGE | 5.1.3.8 | *Trichormus variabilis* | 10 |
| N-acetylneuraminate lyase | NAL | 4.1.3.3 | *Pasteurella multocida* (strain Pm70) | 11 |
| N-acylneuraminate cytidylyltransferase | CSS | 2.7.7.43 | *Neisseria meningitidis MC58* (serogroup B) | 12 |
| Uridine kinase | UDK | 2.7.1.48 | *Escherichia coli* (strain K12) | 13 |
| Uridine monophosphate kinase | URA6 | 2.7.4.47 | *Arabidopsis thaliana* | 14 |
| Polyphosphate kinase 3 | PPK3 | 2.7.4.1 | *Ruegeria pomeroyi* (strain ATCC 700808 / DSM 15171 / DSS-3) | 15 |
| Inorganic diphosphatase | PPA | 3.6.1.1 | *Pasteurella multocida* (strain Pm70) | 16 |
| 2-domain polyphosphate kinase 2 | 2D-PPK2 | 2.7.4.1 | *Pseudomonas aeruginosa* | 17 |
| 1-domain polyphosphate kinase 2 | 1D-PPK2 | 2.7.4.1 | *Pseudomonas aeruginosa* | 18 |

## Claims

1. A method for producing an α-sialyl-β-D-galactoside saccharide, comprising
A) providing a solution comprising a β-D-galactoside saccharide, a sialic acid donor, and
an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
B) mixing said solution and said enzyme and reacting a resulting solution to produce the α-sialyl-β-D-galactoside saccharide.

2. The method according to claim 1, wherein the α-sialyl-β-D-galactoside saccharide is an α-sialyl-(2→6)-β-D-galactoside saccharide and the enzyme with β-galactoside α-sialyltransferase activity is an enzyme with β-galactoside α-2,6-sialyltransferase activity consisting of an amino acid sequence as set forth in SEQ ID NOs: 4 - 5 or an amino acid sequence with at least 80% identity to SEQ ID NOs: 4 - 5.

3. The method according to claim 1, wherein the α-sialyl-β-D-galactoside saccharide is an α-sialyl-(2→3)-β-D-galactoside saccharide and the enzyme with β-galactoside α-sialyltransferase activity is an enzyme with β-galactoside α-2,3-sialyltransferase activity consisting of an amino acid sequence as set forth in SEQ ID NOs: 6 - 9 or an amino acid sequence with at least 80% identity to SEQ ID NOs: 6-9.

4. The method according to any one of claims 1 to 3, wherein the enzyme with β-galactoside α-sialyltransferase activity does not catalyse the hydrolysis of cytidine 5'-monophospho-*N*-acetyl-neuraminic acid to cytidine and *N*-acetyl-neuraminic acid.

5. The method according to any one of claims 1 to 4, wherein the enzyme with β-galactoside α-sialyltransferase activity consists of an amino acid sequence as set forth in SEQ ID NO: 4, 7, and 8.

6. The method according to any one of claims 1 to 5, wherein the β-D-galactoside saccharide is a glycopeptide, a glycoprotein, a glycolipid, a glycan or a human milk oligosaccharide (HMO).

7. The method according to any one of claims 1 to 6, wherein the α-sialyl-β-D-galactoside saccharide is a sialylated human milk oligosaccharide selected from 6'-siallylactose, 3'-siallylactose, sialyllacto-N-tetraose a (LSTa), sialyllacto-N-neotetraose d (LSTd), sialyllacto-N-neotetraose c (LSTc), sialyllacto-N-tetraose b (LSTb)and disialyllacto-N-tetraose (DSLNT).

8. The method according to any one of claims 1 to 6, wherein the β-D-galactoside saccharide is a glycoprotein comprising A2G2 (GlcNAc₂Man₃GlcNAc₂Gal₂).

9. The method according to any one of claims 1 to 6, wherein the α-sialyl-β-D-galactoside saccharide is a sialylated monoclonal antibody.

10. The method according to any one of claims 1 to 9, wherein the sialic acid donor is a sialic acid nucleotide, preferably CMP-Neu5Ac.

11. The method according to any one of claims 1 to 10, wherein the method is an *in vitro* method and the enzyme with β-galactoside α-sialyltransferase activity is an isolated enzyme.

12. The method according to any one of claims 1 to 11, wherein the solution has a pH between 6 and 9 and/or wherein step B) is carried out at a reaction temperature between 5°C and 50°C.

13. The method according to any one of claims 1 to 12, wherein step A') is performed instead of step A)
A') providing a solution comprising a β-D-galactoside saccharide, , *N*-acetyl-D-glucosamine, pyruvate, polyphosphate, catalytic amounts of i) cytidine 5'-monophosphate, ii) cytidine 5'-diphosphate or iii) cytidine 5'-triphosphate, and catalytic amounts of i) adenosine 5'-monophosphate, adenosine 5'-diphosphate, or adenosine 5'-triphosphate, and
a set of enzymes comprising an N-acylglucosamine 2-epimerase (AGE, EC 5.1.3.8), an N-acetylneuraminate lyase (NAL, EC 4.1.3.3), an N-acetylneuraminate cytidylyltransferase (CSS, EC 2.7.7.43), optionally a uridine kinase (UDK, EC 2.7.1.48), a uridine monophosphate kinase (URA6, EC 2.7.4.22), a polyphosphate kinase 3 (PPK3, EC2.7.4.1), and an enzyme with β-galactoside α-sialyltransferase activity consisting of an amino acid sequence as set forth in SEQ ID NOs: 4 - 9 or an amino acid sequence with at least 80% identity to SEQ ID NOs: 4 - 9;
and step B) comprises
B) mixing said solution and said set of enzymes together with the β-galactoside α-sialyltransferase and reacting a resulting solution to produce the α-sialyl-β-D-galactoside saccharide,
wherein the uridine kinase (UDK), if present, transfers *in situ* the cytidine to a cytidine 5'-monophosphate (CMP).

14. The method according to claim 13, wherein the N-acylglucosamine 2-epimerase (AGE) comprises an amino acid sequence as set forth in SEQ ID NO: 10; the N-acetylneuraminate lyase (NAL) comprises an amino acid sequence as set forth in SEQ ID NO: 11; the N-acylneuraminate cytidylyltransferase (CSS) comprises an amino acid sequence as set forth in SEQ ID NO: 12; the optional uridine kinase (UDK) comprises an amino acid sequence as set forth in SEQ ID NO: 13; the uridine monophosphate kinase (URA6) comprises an amino acid sequence as set forth in SEQ ID NO: 14; and the polyphosphate kinase 3 (PPK3) comprises an amino acid sequence as set forth in SEQ ID NO: 15 and wherein the set of enzymes is co-immobilized together with the β-galactoside α-sialyltransferase on a solid support.

15. Sialyltransferases of the SEQ ID NO. 4, SEQ ID NO. 5, SEQ ID NO. 6, SEQ ID NO. 7, SEQ ID NO. 8 and SEQ ID NO. 9.
